# EUROPÄISCHE PATENTSCHRIFT

(11) **EP 4 704 937 B1**
(45) Veröffentlichungstag und Bekanntmachung des Hinweises auf die Patenterteilung: **05.08.2026**
(21) Anmeldenummer: 24801491.2
(22) Anmeldetag: 04.11.2024
(51) Int. Cl.: A61M 1/00, A61P 11/00, A61M 1/16, A61M 1/36

(54) **VORRICHTUNG ZUM ENTERALEN AUFNEHMEN VON CO2, SOWIE DEMENTSPRECHENDES SET**
DEVICE FOR THE ENTERAL ABSORPTION OF CO2, AND CORRESPONDING SET
DISPOSITIF D'ABSORPTION ENTÉRALE DE CO2, ET ENSEMBLE CORRESPONDANT

(30) Priorität: 03.11.2023 DE 102023130381
(43) Veröffentlichungstag der Anmeldung: 11.03.2026
(73) Patentinhaber: O11 biomedical GmbH, 52074 Aachen (DE)
(72) Erfinder: JOCKENHÖVEL, Stefan, 52074 Aachen (DE); RAMAKERS, Richard, 6267CG Cadier en Keer (NL); SIEBERT, Stefan, 52066 Aachen (DE); CORNELISSEN, Christian, 52072 Aachen (DE); KNIEBS, Caroline, 4700 Eupen (BE)
(74) Vertreter: Viering, Jentschura & Partner mbB Patent- und Rechtsanwälte
(86) Internationale Anmeldenummer: PCT/EP2024/080976
(87) Internationale Veröffentlichungsnummer: WO 2025/093759

(56) Entgegenhaltungen:
- DE-A1- 102021 112 959
- US-A- 4 671 287
- US-A1- 2022 040 393

## Beschreibung

Die vorliegende Erfindung betrifft eine Vorrichtung zur kontrollierten *in* vivo-Aufnahme von CO₂ umfassend einen Katheter zur Verwendung im Darmtrakt aufweisend eine gaspermeable Membran und ein CO₂-aufnehmendes Trägermedium. Die Erfindung betrifft zudem ein Set, das diese Vorrichtung umfasst. Weiterhin betrifft die Erfindung die Vorrichtung oder das Set als geeignet zur Verwendung in der Prophylaxe oder Behandlung von Erkrankungen des respiratorischen Systems oder zur Verwendung in der Induktion einer Hypokapnie in einem Patienten.

### Hintergrund der Erfindung

Es gibt zahlreiche respiratorische Erkrankungen, die mit einem erhöhten Gehalt an CO₂ im Blut einhergehen, was auch als Hyperkapnie bezeichnet wird. Ursache einer Hyperkapnie ist meist eine Störung der Lungenventilation (früher respiratorische Globalinsuffizienz genannt), die aufgrund alveolärer Hypoventilation oder Versagen der Atempumpe entsteht. Ein hyperkapnisches Atemversagen kann beispielsweise bei einer Verschlechterung der chronisch-obstruktiven Lungenerkrankung (COPD) auftreten. Die Hyperkapnie entsteht aber auch durch eine metabolische Alkalose oder durch Einatmung stark kohlendioxidhaltiger Luft (ab 8 bis 10 Volumenprozent kommt es zu einer Kohlendioxidvergiftung). Mediziner sprechen auch von einem hyperkapnischen Versagen der Atmung, d.h. einer Kohlendioxid-Vergiftung bei gleichzeitigem Sauerstoffmangel im Blut. Typische Anzeichen einer Hyperkapnie sind Atemnot, Unruhe aber auch Schläfrigkeit, Kopfschmerzen, Verwirrtheit, Blaufärbung der Haut und ein erhöhter Puls.

Zur Behandlung eines hyperkapnischen respiratorischen Versagens der Atmung kann zur Unterstützung der Atemarbeit eine maschinelle Beatmung des Patienten erfolgen, etwa mit einer druckunterstützten nichtinvasiven Beatmung. Eine solche Behandlung ist geräte- und kostenintensiv und mit zahlreichen Risiken verbunden, wie beispielsweise Schädigung der Lunge durch Druck, Lungenentzündung, Steigerung des Drucks im Brustraum oder Verschlechterung einer Herzschwäche. Darüber hinaus ist diese Behandlung äußerst belastend für den Patienten.

Die Hyperkapnie kann jedoch auch im Zuge von intensivmedizinischen Behandlungen von Kindern und Erwachsenen auftreten, wenn erhöhte CO₂-Partialdrücke zugunsten einer schonenderen Beatmung in Kauf genommen werden (auch als "permissive Hyperkapnie" bezeichnet).

Daher besteht ein Bedarf nach alternativen Verfahren zur Behandlung der Hyperkapnie. So gibt es experimentelle Ansätze, durch die orale Gabe von CO₂-Absorbern unter Ausnutzung der großen inneren Oberfläche des menschlichen Verdauungsapparats den CO₂-Gehalt im Blut zu reduzieren. US 2022/0040393 A1 offenbart eine Vorrichtung aus dem Stand der Technik.

Die WO 2020/035163 A1 offenbart hierzu eine Zusammensetzung aus einem CO₂ Absorbens oder Adsorbens mit einer polymeren Beschichtung aus einem SilikonKautschuk hergestellt aus Flüssig-Silikonkautschuk oder mit einer polymeren Beschichtung aus einem Zellulose-Derivat. Sie lehrt hierzu die Kautschukbeschichtung durch Tauchbeschichtung mit Flüssig-Silikonkautschuk und anschließendem Verpressen zu Tabletten.

Die WO 2020/035163 A1 offenbart die Verkapselung eines pulverförmigen CO₂-Absorbers in einer Kapsel aus regenerierter Cellulose.

Weiterer Stand der Technik kann der WO 2005/097075 A2, der US 2015/245999 A1, der US 2017/319617 A1 oder der WO 2023/072929 A1 entnommen werden.

Der vorliegenden Erfindung liegt die Aufgabe zugrunde, den Stand der Technik zu verbessern oder eine Alternative zu bieten.

### Zusammenfassung der Erfindung

Nach einem ersten Aspekt der vorliegenden Erfindung löst die gestellte Aufgabe eine Vorrichtung zum enteralen Aufnehmen von CO₂, aufweisend einen Katheter zur Verwendung im Darmtrakt,
wobei der Katheter
- einen Einlass und
- einen Auslass für ein CO₂-aufnehmendes Trägermedium und
- eine gaspermeable Membran aufweist, wobei
   o die Membran ein Lumen zur Aufnahme des CO₂-aufnehmenden Trägermediums begrenzt,
   o wobei eine erste, innere Seite der Membran zum Lumen hin gerichtet ist, sodass sie bei mit dem CO₂-aufnehmenden Trägermedium gefüllten Lumen mit dem Trägermedium in Kontakt steht, und
   o eine zweite, äußere Seite der Membran zu einer Umgebung hin gerichtet ist, sodass sie bei Positionierung in einem Darm mit dem Darmmilieu in Kontakt steht,
   o wobei die Membran einen Gasdurchlass von der äußeren zur inneren Seite aufweist, sodass die Membran bei Positionierung im Darmmilieu die Diffusion von Gas aus dem Darmtrakt in das CO₂-aufnehmende Trägermedium erlaubt,
und wobei der Katheter zur Verwendung des CO₂-aufnehmenden Trägermediums eingerichtet ist oder mit dem CO₂-aufnehmenden Trägermedium gefüllt ist.

Weitere Ausführungsformen sind Gegenstand der weiteren unabhängigen und abhängigen Ansprüche.

Begrifflich sei hierzu folgendes erläutert:
Unter einem "Katheter" wird im Rahmen der vorliegenden Erfindung eine flexible Vorrichtung verstanden, die über eine Körperöffnung in den Körper eingeführt werden kann und die Anschlüsse zum Einleiten und Ausleiten eines bevorzugt flüssigen Trägermediums aufweist.

Der Katheter ist entsprechend zur Verwendung des CO₂-aufnehmenden Trägermediums eingerichtet, insofern er bei der therapeutischen Anwendung mit dem CO₂-aufnehmenden Trägermedium gefüllt wird und über Einlass und Auslass einen Austausch von CO2-haltigem Trägermedium mit frischem Trägermedium ermöglicht. In einer Ausführungsform ist der Katheter mit dem CO₂-aufnehmenden Trägermedium gefüllt.

Der erfindungsgemäße Katheter ist für die Verwendung im Darmtrakt vorgesehen und damit in der Größe und die Beschaffenheit an den Darmtrakt eingerichtet. Er wird entsprechend rektal durch den Anus eingeführt und im Lumen des Darms positioniert. Bevorzugt wird er hierbei im *Colon descendens* und/oder im *Colon sigmoideum* positioniert, also zwischen der *Flexura coli sinestra* und dem Rectum.

Unter dem Darmmilieu wird dasjenige Milieu verstanden, das den im Darmtrakt positionierten Katheter umgibt. Dieses umfasst das Lumen des Darmtrakts und bei einem Katheter, der (wenn auch nur temporär) mit mindestens einem Teil der Membran die Darmwand kontaktiert, entsprechend auch die Darmwand als CO₂-haltiges Gewebe.

Die Erfindung weist mehrere Vorteile gegenüber dem Stand der Technik auf.

Durch den Katheter mit der umhüllenden Membran können große Mengen an CO₂-aufnehmenden Trägermedium im Körper bereitgestellt werden. In Verbindung mit der großen Oberfläche des End/Dickdarms kann dadurch sehr schnell relevante Mengen an CO₂ aus dem Körper abgeführt werden, was den Katheter besonders für eine Behandlung von Intensiv- und Risikopatienten qualifiziert.

Durch die vollständige Umhüllung mit der Membran liegt eine geschlossene und sichere Ummantelung vor, so dass ein breites Spektrum an CO₂-aufnehmenden Trägermedien verwendet werden kann und auch Medien, die mit Nebenwirkungen einhergehen, in sicherer Weise eingesetzt werden können.

Der Fachmann kann durch Kombination mehrere Parameter, wie die Wahl des CO₂-Trägermediums, dessen Konzentration und die Flussrate, die erfindungsgemäße Vorrichtung gezielt an den jeweiligen therapeutischen Verwendungszweck anpassen. Zudem erlaubt gerade die Veränderung der Flussrate des Trägermediums eine gezielte Anpassung an die Bedürfnisse des Patienten und kann auch innerhalb einer Behandlungssitzung durchgeführt werden.

Die Ausgestaltung als Katheter ist einer großtechnischen Herstellung zugänglich.

Die vorliegende Vorrichtung mit dem Katheter kann aufgrund ihrer enteralen Gabe breit zur Therapie respiratorischer Erkrankungen eingesetzt werden, ohne den respiratorischen Trakt zu beeinträchtigen oder zu belasten.

Es ergibt sich eine innovative und nebenwirkungsarme Therapiemöglichkeit, die gerade bei Intensivpatienten oder Risikopatienten angewendet werden kann.

### Die Erfindung im Einzelnen

Erfindungsgemäß weist der Katheter einen Einlass und einen Auslass für ein CO₂-aufnehmendes Trägermedium auf, wobei es sich um zwei separate Zugänge handelt.

In einer alternativen Ausführungsform, welche nicht Teil der beanspruchten Erfindung ist, kann der Einlass auch als Auslass fungieren. In dieser Ausführungsform kann das CO₂-aufnehmende Trägermedium in einem ersten Schritt durch den Zugang in den Katheter eingebracht werden, um dann, nach Aufnahme von CO₂ durch denselben Zugang wieder aus dem Katheter ausgeführt zu werden.

Gemäß der beanspruchten Erfindung ist das CO₂ -aufnehmende Trägermedium eine Flüssigkeit, in welcher eine CO₂ -absorbierende Substanz dispergiert ist.

Vorteilhafterweise kann bei der erfindungsgemäßen Vorrichtung vorgesehen sein, dass die gaspermeable Membran wasserundurchlässig ist und/oder einen "Molecular weight cutoff" (MWCO) von weniger als 200 Dalton aufweist.

Eine wasserundurchlässige Membran verhindert eine Diffusion von Wassermolekülen aus dem Darm in den Katheter und umgekehrt aus dem Katheter in den Darm, wodurch der Wasserhaushalt des Darms unverändert bleibt und einer möglichen Obstipation bzw. Diarrhoe wird vorgebeugt.

In einer Ausführungsform ist die gaspermeable Membran zudem undurchlässig für Wasserdampf. Bei der bevorzugten Verwendung eines flüssigen Trägermediums ist ein Anfeuchten des Trägermediums nicht erforderlich.

Der Begriff der "Wasserundurchlässigkeit" ist im Rahmen der vorliegenden Anmeldung synonym mit dem Begriff "Wasserdichte". Die sogenannte Wasserdichte der gaspermeablen Membran wird anhand der Wassersäule gemessen, unter deren Druck das Material beginnt, Wasser durchzulassen. Ab einem Wert von 1.300 mm aufwärts ist eine Membran laut DIN-Norm EN 343:2019-06 wasserundurchlässig.

In einer bevorzugten Ausführungsform ist die gaspermeable Membran des Katheters so ausgestaltet, dass sie bei der *in* vivo-Anwendung eine Freisetzung des CO₂-aufnehmenden Trägermediums bzw. des CO₂-Absorbers im Wesentlichen verhindert. Hierbei bedeutet "im Wesentlichen", dass bei der *in* vivo-Anwendung des Katheters höchstens 5 Gew.%, vorzugsweise höchstens 4 Gew.%, bevorzugt höchstens 3 Gew.%, weiter bevorzugt höchstens 1 Gew.% des CO₂-aufnehmenden Trägermediums bzw. des CO₂-Absorbers aus dem Katheter freigesetzt werden.

Viele CO₂-aufnehmende Trägermedien bzw. CO₂-Absorber führen bei der Freisetzung im Gastrointestinaltrakt zu unerwünschten Nebenwirkungen. Bei Calciumhydroxid als starker Base würde die Freisetzung zu einer starken pH-Wert-Erhöhung führen. Bei Magnesiumhydroxid könnte eine zu starke Freisetzung mit Muskelschwäche und Diarrhoe einhergehen.

Es handelt sich bei dem CO₂-absorbierenden Katheter erfindungsgemäß um eine Vorrichtung, die ausschließlich der Absorption von CO₂ aus dem Organismus dient, eine Freisetzung des CO₂-aufnehmenden Trägermediums bzw. des CO₂-Absorbers, sei es als CO₂-freies Edukt oder CO₂-haltiges Produkt soll möglichst verhindert werden. In diesem Sinne handelt es sich bei der gaspermeablen Membran des Katheters um eine semipermeable bzw. selektiv permeable Barriere, die zwar CO₂ aus dem Organismus hindurchlässt, um eine Absorption am CO₂-Absorber im Inneren des Katheters zu erlauben, aber impermeabel für den CO₂-Absorber und bevorzugt auch impermeabel für dessen Absorptionsprodukt(e) ist.

Entsprechend ist die gaspermeable Membran des Katheters impermeabel für das CO₂-aufnehmende Trägermedium bzw. den CO₂-Absorber und bevorzugt dessen Absorptionsprodukt(e) und dies auch für die Dauer der *in* vivo-Anwendung.

In einer alternativen bevorzugten Ausführungsform ist die gaspermeable Membran des Katheters so ausgestaltet, dass sie bei der *in* vivo-Anwendung eine Freisetzung des CO₂-aufnehmenden Trägermediums bzw. des CO₂-Absorbers im Gastrointestinaltrakt so weit verhindert, dass die Konzentration des CO₂-aufnehmenden Trägermediums bzw. des CO₂-Absorbers im Darmlumen weniger als ein 1mM, bevorzugt weniger als 100 M, weiterhin bevorzugt weniger als 10µM und insbesondere bevorzugt weniger als 1 µM beträgt.

Diese minimierte Freisetzung kann auch anhand einer *in* vitro-Untersuchung bestimmt werden. Sowohl das europäische Arzneibuch (Ph.Eur.) als auch das amerikanische Arzneibuch (USP) definieren für *in vitro* Wirkstofffreisetzungs-Untersuchungen genaue Vorgehensweisen und Apparaturen. Das Europäische Arzneibuch benennt die Apparatur entsprechend der Arzneiform. So werden im Kapitel 2.9.3 "Wirkstofffreisetzung aus festen Arzneiformen" beispielsweise die folgenden definierten Apparaturen aufgeführt: Drehkörbchen-Apparatur (Rotating Basket, USP); Blattrührer-Apparatur (Rotating Paddle, USP); oder Durchflusszelle (Flow-through cell, USP). Dem Fachmann sind Reaktionstemperaturen und Puffer bekannt, die eine Freisetzung des CO₂-aufnehmenden Trägermediums bzw. des CO₂-Absorbers im Gastrointestinaltrakt simulieren. Wie die Untersuchung durchgeführt werden muss, richtet sich nach der Wirkstofffreisetzung der Darreichungsform und er wird hier die verzögerte Freisetzung auswählen.

Die vorliegende Formulierung weist eine selektiv permeable Membran auf, wobei die selektive Permeabilität darin besteht, dass die Membran einerseits permeabel für CO₂ ist (und damit das CO₂ aus dem Gastrointestinaltrakt durch die Membran zu dem CO₂-aufnehmenden Trägermedium bzw. dem CO₂-Absorber gelangen kann und dort gebunden werden kann), und andererseits impermeabel für das CO₂-aufnehmende Trägermedium bzw. den CO₂-Absorber ist (sei es als CO₂-freies Edukt oder CO₂-haltiges Produkt). Die selektive Permeabilität der vorliegenden Membran bleibt während des Verweilens des Katheters im Gastrointestinaltrakt bestehen. Eine physiologisch wirksame Menge an CO₂ wird aus dem Gastrointestinaltrakt des Menschen entfernt.

Der Katheter mit seiner gaspermeablen Membran einer beliebigen Ausführungsform der Erfindung ist vorzugsweise ausreichend robust, um in der Umgebung der Verwendung fortzubestehen, beispielsweise, um im GI-Trakt oder einen dafür repräsentativen *in vitro* Assay für pharmazeutische Anwendungen zu persistieren, ohne dass ein derartiger Katheter erheblich zersetzt wird und/oder vorzugsweise ohne dass die physikalischen Charakteristika und/oder Leistungscharakteristika des Katheters erheblich verschlechtert werden. In bevorzugten Ausführungsformen wird die gaspermeable Membran im Wesentlichen nicht zersetzt und/oder besitzt physikalische Charakteristika und/oder Leistungscharakteristika, die unter physiologischen Bedingungen des GI-Trakts (oder *in* vitro-Darstellungen oder Nachahmungen hierfür) während eines Zeitraums für das Verweilen in der Umgebung von Interesse, wie zum Beispiel im Gastrointestinaltrakt, im Wesentlichen nicht verschlechtert wird.

Vorzugsweise bindet das CO₂-aufnehmende Trägermedium bzw. der CO₂-Absorber das CO₂ und halten das CO₂ für einen bedeutsamen Zeitraum in der Umgebung von Interesse fest. Zum Beispiel kann der Katheter in Anwendungen, die das Binden von CO₂ im Gastrointestinaltrakt beinhalten, CO₂ in den Bereichen des Gastrointestinaltrakts binden, die eine relativ hohe Konzentration an CO₂ aufweisen. Derartig gebundenes CO₂ bleibt vorzugsweise am CO₂-aufnehmenden Trägermedium bzw. am CO₂-Absorber gebunden und wird aus dem Körper in einer ausreichenden Menge ausgeschieden, so dass ein therapeutisch vorteilhafter Effekt auftritt. Aus einer alternativen Perspektive setzt der Katheter das gebundene CO₂ in der Umgebung von Interesse, zum Beispiel im Gastrointestinaltrakt, nicht in signifikanter Weise frei, bevor ein gewünschter therapeutischer Effekt erzielt wird. Die hierin beschriebenen Katheter können eine signifikante Menge an CO₂ festhalten. Der Begriff "signifikante Menge", wie er hierin verwendet wird, soll nicht bedeuten, dass die gesamte Menge an gebundenem CO₂ festgehalten wird. Es ist bevorzugt, dass zumindest ein Großteil des gebundenen CO₂ festgehalten wird, so dass ein therapeutisch relevanter Effekt auftritt.

Der Retentionszeitraum liegt im Allgemeinen bevorzugt während der Zeit, in der der Katheter in der Umgebung von Interesse verwendet wird. Für Anwendungen, die das Binden von CO₂ im Gastrointestinaltrakt beinhalten, ist diese Zeit beispielsweise ein Zeitraum, der für einen therapeutisch vorteilhaften Effekt ausreichend ist. In der Ausführungsform, in der Katheter verwendet wird, um CO₂ zu binden und aus den Gastrointestinaltrakt zu entfernen, kann der Retentionszeitraum im Allgemeinen die Verweilzeit der Zusammensetzung im Gastrointestinaltrakt und stärker bevorzugt die mittlere Verweilzeit im Dünndarm und Dickdarm sein.

Vorteilhafterweise ist die permeable Selektivität des Katheters der Erfindung ausreichend dauerhaft, um einen nützlichen Effekt, beispielsweise eine Behandlung einer Erkrankung des respiratorischen Systems zu bewirken. Die dauerhafte Selektivität (zum Beispiel die dauerhafte selektive Permeabilität) der Kathetermembran ist für das Binden von CO₂ im Gastrointestinaltrakt in besonderer Weise vorteilhaft.

Die Katheter der Erfindung sind vorzugsweise ausreichend robust, um in der Umgebung der beabsichtigten Verwendung fortzubestehen. In einer Anwendung sind die Katheter mit ihrer gaspermeablen Membran beispielsweise ausreichend robust, um im gastrointestinalen System zu verweilen (oder in einem dafür repräsentativen in vitro-Assay zu bestehen), ohne dass ein derartiger Katheter, bzw. dessen Membran wesentlich zersetzt wird. In bevorzugten Ausführungsformen ist die gaspermeable Membran des CO₂-Absorbers unter physiologischen Bedingungen des Gastrointestinaltrakts (oder in *in vitro-*Darstellungen oder Nachahmungen davon) während eines Zeitraums des Verweilens im Gastrointestinaltrakt im Wesentlichen robust (sie wird beispielsweise nicht zersetzt, perforiert oder getrennt und/oder entschichtet). Beispielsweise werden der CO₂-Absorber und Membran unter *in* vitro-Bedingungen im Wesentlichen nicht zersetzt, wobei diese Bedingungen bevorzugt ausgewählt sind aus der Gruppe, bestehend aus (ii) einer wässrigen Lösung mit einem pH von (etwa) 8 in einem Zeitraum von (etwa) 10 Stunden, (iii) einer wässrigen Lösung mit einem pH von (etwa) 6 über einen Zeitraum von (etwa) 20 Stunden sowie Kombinationen davon, jeweils bei einer Temperatur von (etwa) 37°C unter Rühren.

In einigen Ausführungsformen kann das CO₂-aufnehmende Trägermedium bzw. bevorzugt der partikuläre CO₂-Absorber robust sein - in Bezug auf andere Gesichtspunkte zusätzlich dazu, dass sie nicht zersetzt werden, beispielsweise einschließlich in Bezug auf physikalische Charakteristika und/oder Leistungscharakteristika. Physikalische Charakteristika können die Partikelgröße, die Partikelgrößenverteilung und/oder die Oberflächeneigenschaften, beispielsweise unter Verwendung von Mikroskopen, zum Beispiel von Elektronenmikroskopen und/oder konfokalen Mikroskopen visuell beurteilt, beinhalten. Leistungscharakteristika können eine spezifische Bindungsfähigkeit, Selektivität (zum Beispiel selektive Permeabilität) und Dauerhaftigkeit bzw. Persistenz beinhalten.

Einige bevorzugte *in vitro*-Assays, die in Zusammenhang mit der Bestimmung der Robustheit verwendet werden können, zum Beispiel für die Zwecke der Modifikation bzw. Optimierung eines Katheters in dieser Hinsicht, beinhalten die vorab aufgeführten *in vitro* Wirkstofffreisetzungs-Untersuchungen, wobei hier das CO₂-aufnehmende Trägermedium bzw. der CO₂-Absorber als "Wirkstoff" gerade nicht freigesetzt werden soll.

In einigen Ausführungsformen kann die Membran andere Eigenschaften verleihen, die sich auf die Robustheit beziehen, beispielsweise eine ausreichende Beständigkeit, um mechanischen Kräften oder Spannungen im Zusammenhang mit einer Flüssigkeitsfüllung unter Druckbeaufschlagung im Zusammenhang mit der therapeutischen Anwendung standzuhalten.

In Ausführungsformen der Erfindung kann die Membran das CO₂-aufnehmende Trägermedien bzw. den CO₂-Absorber vor der äußeren Umgebung, zum Beispiel dem Gastrointestinaltrakt, schützen.

Vorteilhafterweise kann bei der Erfindung vorgesehen sein, dass das CO₂-aufnehmende Trägermedium eine Flüssigkeit ist, d.h. das Trägermedium befindet sich im Betrieb (bei Standardbedingungen) im flüssigen Aggregatzustand. Aufgrund der Verwendung einer Flüssigkeit als Trägermedium ist der Ab- und Zutransport von Substanzen wesentlich kontrollierter und effizienter durchführbar.

Aufgrund der Verwendung einer Flüssigkeit als CO₂-aufnehmendes Trägermedium ist die Membran der Vorrichtung eine für Flüssigkeiten geeignete Membran, d.h. zum Austausch von Substanzen zwischen zwei Flüssigkeiten konfiguriert und eingerichtet. Auch wenn mit einer solchen Membran auch in gewissem Ausmaß ein Austausch von Substanzen zwischen einer Flüssigkeit und einem Gas erzielt werden könnte oder kann, ergibt sich aus der Konfiguration der Membran insgesamt, vor allem aus der vorgesehenen Kontaktfläche und/oder aus der mechanischen Stabilität, dass sie in einer Ausführungsform nicht für die Verwendung mit einem gasförmigen Trägermedium eingerichtet ist.

Durch die Verwendung geeigneter Trägerflüssigkeiten und eine verstärkte Zufuhr dieser Flüssigkeit kann eine höhere CO₂-Aufnahme durch die Membran erzielt werden und die nötige Membranoberfläche verringert werden, was zu einer verringerten Druckdifferenz im Katheter führt und damit auch eine Verkleinerung der Fördereinrichtung ermöglicht.

In einer Ausführungsform welche nicht Teil der beanspruchten Erfindung ist, ist das CO₂-aufnehmende Trägermedium ausgewählt aus der Gruppe bestehend aus:
(a) einer Trägerflüssigkeit, in welcher CO₂ löslich ist, wobei die CO₂-Löslichkeit bei 20 °C und 1 Atm. mindestens 85 ml CO₂/100 ml Trägerflüssigkeit beträgt;
(b) einer Flüssigkeit, in welcher eine CO₂-adsorbierendes Material gelöst oder dispergiert vorliegt;
(c) einer Flüssigkeit, in welcher ein CO₂-umsetzendes Enzym wie Carboanhydrase gelöst ist.

In einer Ausgestaltung kann die Trägerflüssigkeit hierbei selber als CO₂-aufnehmendes Trägermedium dienen, insofern es sich um eine Trägerflüssigkeit, in welcher CO₂ löslich ist, und wobei die CO₂-Löslichkeit bei 20°C und 1 Atm. mindestens 85 ml CO₂/100 ml Trägerflüssigkeit beträgt. Die CO₂-Löslichkeit bei 20°C und 1 Atm. kann hierbei mindestens 90, 95, 100, 110, 120, 130, 140, 150, 160, 170, 180, 190, 200, 210, 220, 230, 240 oder 250 ml CO₂/100 ml Trägerflüssigkeit betragen.

Bei der Trägerflüssigkeit mit CO₂-Löslichkeit handelt es sich um eine physikalische Absorption. Der Begriff der "Absorption" beschreibt den Prozess der Aufnahme oder des "Lösens" des Kohlendioxids in einer anderen Phase. Hierbei handelt es sich nicht um eine Anlagerung an der Oberfläche (Adsorption), sondern um eine Aufnahme in das freie Volumen der absorbierenden Phase. Bei der physikalischen Absorption wird das Kohlendioxid als Gas in einem Lösungsmittel aufgelöst. Es findet eine Vermischung ohne chemische Reaktion statt.

Die Aufnahmekapazität für CO₂ im Blut beträgt ca. 20 ml/100ml. Dabei liegt Kohlendioxid im Blut in zwei Formen vor: Ein kleinerer Teil ist als freies CO₂ physikalisch im Blutplasma gelöst. Der größere Teil ist im Plasma und den Erythrozyten als Hydrogencarbonat (Bicarbonat) gelöst, beziehungsweise kommt in den Erythrozyten auch an Hämoglobin gebunden vor (sog. Carbaminohämoglobin).

In einer alternativen Ausführungsform ist das CO₂-aufnehmende Trägermedium eine Flüssigkeit, in welcher ein CO₂-adsorbierendes Material gelöst oder dispergiert vorliegt. Als "Adsorption" wird vorliegend die Anreicherung des Kohlendioxids an der Oberfläche eines Festkörpers bezeichnet.

Bei der Vorrichtung kann vorteilhafterweise vorgesehen sein, dass das CO₂-adsorbierende Material ausgewählt ist aus der Gruppe bestehend aus Aktivkohle, einem Molekularsieb wie Zeolith, Silika und metallorganische Gerüstverbindungen (MOFs).

Aktivkohle ist eine feinkörnige Kohle mit großer innerer Oberfläche, die zwischen 300 und 2000 m²/g Kohle betragen kann. Der Begriff "Molekularsieb" ist vorliegend die funktionelle Bezeichnung für natürliche und synthetische Zeolithe oder andere Stoffe, die eine hohe Adsorptionskapazität für das Kohlendioxid-Gas haben. Neben Zeolithen gibt es beispielsweise auch Kohlenstoffmolekularsiebe (engl. "carbon molecular sieve" oder "molecular sieving carbon"). Die Molekularsiebe weisen eine große innere Oberfläche (600-700 m²/g) auf und haben einheitliche Porendurchmesser, die in der Größenordnung der Durchmesser von Molekülen liegen. Metallorganische Gerüstverbindungen (englisch metalorganic frameworks, MOFs) sind gemäß der vorliegenden Anmeldung definiert als mikroporöse Materialien, die aus anorganischen Baueinheiten, den sogenannten Inorganic building units (IBUs, englisch für "anorganische Baueinheiten") und organischen Molekülen als Verbindungselementen (englisch linkers) zwischen den anorganischen Baueinheiten aufgebaut sind. MOFs sind Koordinationsnetzwerke mit einem offenen Gerüst, welches Poren enthält. Die Poren der dreidimensionalen Strukturen sind nach der Synthese meist mit Gastmolekülen (z. B. Lösungsmittel oder nicht umgesetzte Linker) gefüllt. Durch die Entfernung der Gastmoleküle (z. B. durch Ausheizen, im Vakuum oder durch Kombination von beidem) können die Poren wieder für die Aufnahme von Gasen wie CO₂ zugänglich gemacht werden.

Gemäß der beanspruchten Erfindung ist das CO₂-aufnehmende Trägermedium eine Flüssigkeit, in welcher eine CO₂-absorbierende Substanz dispergiert vorliegt. Unter der Absorption ist hierbei eine chemische Absorption zu verstehen, bei der das Kohlendioxid eine chemische Reaktion mit dem "Lösungsmittel" bzw. Absorbens eingeht, sodass sich ein Produktstoff bildet.

So kann das CO₂ beispielsweise mit einer anorganischen Hydroxid-Verbindung wie Calciumhydroxid gemäß der folgenden Reaktionsgleichung (I) zu Calciumcarbonat reagieren. Als Nebenprodukt der Reaktion entsteht Wasser:

(I) Ca(OH)₂(aq) + CO₂(g) → CaCO₃(s) + H₂O(l)

In einer alternativen Ausführungsform handelt es sich bei dem CO₂-aufnehmenden Trägermedium um eine Flüssigkeit, in welcher ein CO₂-umsetzendes Enzym wie Carboanhydrase gelöst ist. Durch dieses Enzym kann das durch die Membran diffundierende gasförmige CO₂ gemäß der folgenden Reaktionsgleichung (II) in Bicarbonat und Wasserstoffionen umgesetzt werden:

(II) CO₂ + 2 H₂O 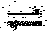 H₂CO₃ + H₂O 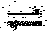 H₃O⁺ + HCO₃⁻

Beim Menschen existieren mehr als 10 verschiedene Isoformen der α-Carboanhydrase, die systematisch mit römischen Zahlen nummeriert werden (CA-I, CA-II, CA-III usw.). Das Zinkion ist als aktives Zentrum für die eigentliche Aktivität des Enzyms verantwortlich. Es ist im Proteingerüst an drei von Histidin abgeleitete Imidazolreste gebunden. Die vierte Koordinationsstelle wird von einem Hydroxo-Liganden (OH-) besetzt. Das Zink der Carboanhydrasen ist daher tetrakoordiniert. In unmittelbarer Nähe befindet eine Tasche zur Aufnahme von CO₂.

Es kann bei der Erfindung vorgesehen sein, dass die CO₂-absorbierende Substanz eine anorganische Hydroxidverbindung ist. Als Hydroxidverbindung können sie sich mit dem Kohlendioxid zu den entsprechenden Carbonaten umsetzen. Hierbei ist die Hydroxidverbindung bevorzugt ausgewählt aus Lithiumhydroxid, Natriumhydroxid, Kaliumhydroxid, Calciumhydroxid und Magnesiumhydroxid oder Mischungen hiervon. Besonders bevorzugt ist die Verwendung von Magnesiumhydroxid.

Gemäß einer Ausführungsform besteht die CO₂-absorbierende Substanz im Wesentlichen aus Calciumhydroxid. Das Calciumhydroxid reagiert mit dem Kohlendioxid unter Bildung von pharmazeutisch unbedenklichen Calciumcarbonat. Hierbei bedeutet "im Wesentlichen", dass die CO₂-absorbierende Substanz höchstens 5 Gew.%, vorzugsweise höchstens 4 Gew.%, bevorzugt höchstens 3 Gew.%, weiter bevorzugt höchstens 1 Gew.% andere Komponenten enthält.

In einer vorteilhaften Ausführungsform ist vorgesehen, dass das Calciumhydroxidenthaltende, CO₂-absorbierende Material im Wesentlichen frei von Natrium- und Kaliumhydroxid ist. Hierbei bedeutet "im Wesentlichen", dass das CO₂-absorbierende Material höchstens 4 Gew.%, vorzugsweise höchstens 2 Gew.%, bevorzugt höchstens 1 Gew.%, weiter bevorzugt 0,5 Gew.% an Natrium- und Kaliumhydroxid, und insbesondere bevorzugt kein Natrium- und Kaliumhydroxid enthält.

Gemäß einer besonders bevorzugten Ausführungsform besteht das CO₂-absorbierende Material im Wesentlichen aus Magnesiumhydroxid. Magnesiumhydroxid hat den Vorteil, dass es aufgrund der geringeren Basizität sogar bei einer Ruptur des Katheters mit Materialaustritt keine nennenswerten Nebenwirkungen mit sich bringt.

Das Magnesiumhydroxid reagiert mit dem Kohlendioxid unter Bildung von pharmazeutisch unbedenklichen Magnesiumcarbonat. Hierbei bedeutet "im Wesentlichen", dass das CO₂-absorbierende Material höchstens 5 Gew.%, vorzugsweise höchstens 4 Gew.%, bevorzugt höchstens 3 Gew.%, weiter bevorzugt höchstens 1 Gew.% andere Komponenten enthält.

In einer vorteilhaften Ausführungsform ist vorgesehen, dass das Magnesiumhydroxidenthaltende, CO₂-absorbierende Material im Wesentlichen frei von Natrium- und Kaliumhydroxid ist. Hierbei bedeutet "im Wesentlichen", dass das CO₂-absorbierende Material höchstens 4 Gew.%, vorzugsweise höchstens 2 Gew.%, bevorzugt höchstens 1 Gew.%, weiter bevorzugt 0,5 Gew.% an Natrium- und Kaliumhydroxid, und insbesondere bevorzugt kein Natrium- und Kaliumhydroxid enthält.

Vorteilhafterweise kann bei der Erfindung vorgesehen sein, dass die CO₂-Iösende Flüssigkeit ausgewählt ist aus der Gruppe bestehend aus stark eutektischem Lösungsmittel, ionischer Flüssigkeit und Perfluorcarbon.

Perfluorcarbone (PFCs) sind synthetische, vollständig halogenierte Kohlenstoffverbindungen. Meist sind die Wasserstoffatome durch Fluor ersetzt, aber auch andere Halogene wie Brom oder Chlor kommen vor. Sie können große Mengen an Gasen wie Sauerstoff oder Kohlendioxid aufnehmen und wieder abgeben. Wegen der sehr hohen Kohlenstoff-Fluor-Bindungsenergie sind PFCs chemisch und metabolisch inert; d. h. sie bilden keine toxischen Metabolite. PFCs sind weder hydro- noch lipophil und nicht mischbar mit wässrigen Flüssigkeiten wie Blut. PFCs besitzen im Vergleich zu Blut eine zehnmal höhere Aufnahmekapazität für CO₂ auf und stellen damit eine hocheffiziente Trägerflüssigkeit für CO₂ dar. Weiterhin besitzen PFCs eine niedrige Oberflächenspannung und breiten sich aufgrund ihres hohen Ausbreitungskoeffizienten sehr leicht auf Oberflächen aus. Diese Eigenschaften machen diese Flüssigkeiten besonders geeignet für einen Einsatz mit Hohlfasermembranen.

In einer bevorzugten Ausführungsform ist das Perfluorcarbon ausgewählt aus der Gruppe bestehend aus Perfluordecalin, Perfluordecylbromid, Perfluoroctylbromid, Perfluordichloroctan, Perfluorisobutylcyclohexan, Perfluortributylamin und Perfluormethylcyclohexylpiperidin.

Als CO₂-lösende Flüssigkeit kann auch ein stark eutektisches Lösungsmittel verwendet werden. Als stark eutektische Lösungsmittel (DES von englisch deep eutectic solvents) im Rahmen der vorliegenden Anmeldung werden mehrkomponentige, eutektische Salzschmelzen bezeichnet, deren Schmelzpunkt, wie der von ionischen Flüssigkeiten, nahe oder unter der Raumtemperatur liegt. Beispiele für eutektische Lösungsmittel basieren auf einer Mischung aus quartärer Ammoniumverbindung mit Wasserstoffbrückenbindungsdonoren (z. B. Amin) und einer Carbonsäure.

Ionische Flüssigkeiten (englisch lonic Liquids (IL), sind Salze, deren Schmelztemperatur weniger als 100 °C beträgt. In bevorzugter Weise liegt ihre Schmelztemperatur unter 37°C, weiterhin bevorzugt unter Raumtemperatur (dann als "Room Temperature lonic Liquids" (RTIL) bezeichnet), so dass sie in flüssiger Form im Katheter eingesetzt werden können. Wie alle Salze bestehen sie aus Anionen und Kationen. Durch deren Variation können die physikalisch-chemischen Eigenschaften einer ionischen Flüssigkeit in weiten Grenzen variiert und auf technische Anforderungen hin optimiert werden. Ionische Flüssigkeiten werden vor allem als Lösungsmittel verwendet und auf Grund ihrer strukturellen Vielfalt auch als "designer solvents" bezeichnet. Als CO₂-aufnehmende ionische Flüssigkeit ist beispielsweise das 1-Butyl-3-Propylamino-imidazoliumtetrafluoroborat bekannt, das gemäß des folgenden Reaktionsschemas (III) als anwendungsspezifische ionische Flüssigkeit (TS-IL; Task specific ionic liquid) äquimolare Mengen an CO₂ binden kann:

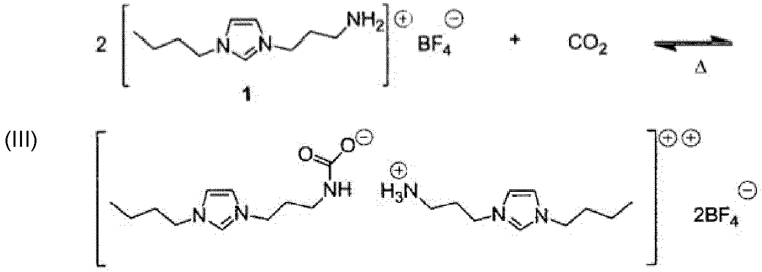

Ein weiteres Beispiel für eine CO₂-speichernde ionische Flüssigkeit ist das 1-Butyl-3-methylimidazoliumhexafluorophosphat (BMIM PF₆). Mit einem Schmelzpunkt von 12°C handelt es sich bei BMIM PF₆ um eine Raumtemperatur-ionische Flüssigkeit (RTIL).

In einer Ausführungsform der Erfindung kann das CO₂-aufnehmende Trägermedium ein Gas oder Gasgemisch sein, das bevorzugt einen CO₂-Partialdruck von weniger als 40 mm Hg aufweist. So kann beispielweise Stickstoff oder Luft als Gasgemisch in den Katheter eingeleitet werden.

Basierend auf einer bevorzugten Verwendung einer Trägerflüssigkeit als Trägermedium ist die gaspermable Membran des Katheters eine für Flüssigkeiten geeignete Membran, d.h. zum Austausch von Substanzen zwischen zwei Flüssigkeiten konfiguriert und eingerichtet.

Je nach Anwendung, d.h. insbesondere in Abhängigkeit von der auszutauschenden Substanz und damit auch in Abhängigkeit von der Trägerflüssigkeit, kann der Aufbau, das Material und die Struktur der Membran entsprechend angepasst sein.

Als Materialien kommen beispielsweise hydrophile oder hydrophilisierte Copolymere und hydrophile Polymermischungen in Frage. Im Einzelnen können Mischungen mit einer oder mehreren Komponenten einer Gruppe bestehend aus Poly(organo)siloxan, Polyethylen wie High-Density-Polyethylen (HDPE) oder Low-Density-Polyethylen (LDPE), Polypropylen, thermoplastisches Polyurethan, Polyester; Polybutylensuccinat; Polybutylenadipat-terephthalat (PBAT), Polyethersulfon (PES), Polyarylethersulfon, Polyacrylethersulfon (PAES), Polyvinylpyrrolidon (PVP), Polymethylmethacrylat (PMMA), Polymethylpenten (PMP), Polyamid (PA), Polyacrylnitril (PAN), Polytetrafluorethylen, Ethylen-Vinylalkohol-Copolymer (EVOH), Cellulose, Cellulosetriacetat (CTA), Cellulosenitrat und Silikon-beschichtetes Polypropylen als Membranmaterial verwendet werden.

In einer Ausführungsform kann die Membran ein Composit aus mikroporöser PE-Außenschicht, PU-Zwischenschicht und mikroporöser PE-Innenschicht sein.

In bevorzugter Weise umfasst die gaspermeable Membran Poly(organo)siloxan, so beispielsweise in Form einer Beschichtung wie eine mit Silikonkautschuk beschichtete PE-Folie oder die Membran besteht in besonders bevorzugter Weise aus Poly(organo)siloxan.

Die Porengröße der gaspermeablen Membran kann zwischen 0,01 µm und 0,1 µm liegen.

Als Membran wird geeigneterweise ein pharmazeutisch unbedenkliches Material, welches bevorzugt ein Polymer ist, verwendet. Damit wird eine nebenwirkungsarme bzw. nebenwirkungsfreie *in* vivo-Anwendung ermöglicht.

Gemäß einer weiteren Ausführungsform kann vorgesehen sein, dass die Membran zur Bereitstellung eines gleitfähigen Katheters eine der folgenden Eigenschaften aufweist:
(a) die Membran besteht aus einem gleitfähigen Polymer;
(b) die Membran weist eine Beschichtung aus einer gleitfähigen Substanz, wie wasserlösliches Gleitgel, PTFE oder Poly(organo)siloxan auf;
(c) die Membran wird vor dem rektalen Einführen mit einem Gleitmittel versehen.

Die Ausgestaltung als gleitfähiger Katheter erleichtert die rektale Applikation und die Positionierung im Darmtrakt und ermöglicht auch die Verwendung von größer dimensionierten Kathetern.

Vorteilhafterweise kann bei der Erfindung vorgesehen sein, dass die Gesamtoberfläche der gaspermeablen Membran, welche die Kontaktoberfläche für die Gasaufnahme bzw. den Gasaustausch bildet, mindestens 0,1 m² beträgt. Die Kontaktoberfläche kann hierbei mindestens 0,2, 0,3, 0,4, 0,5, 0,6, 0,7, 0,8, 0,9 oder 1,0 m² betragen.

Vorteilhafterweise kann bei der Erfindung vorgesehen sein, dass die gaspermeable Membran als Hohlfasermembran ausgebildet ist, wobei die innere Oberfläche der Hohlfasermembran mit dem Trägermedium in Kontakt steht und die äußere Oberfläche der Hohlfasermembran mit dem Darmtrakt in Kontakt steht.

Eine besonders große Kontaktfläche auf kleinem Raum kann erzielt werden, wenn die Membran eine Hohlfasermembran ist. Die eigentliche Kontaktfläche wird dabei durch die Wände der Hohlfasern oder Kapillaren gebildet. Eine Hohlfasermembran kann bis zu 20.000 einzelne Kapillaren oder Hohlfasern umfassen. Der Durchmesser der einzelnen Hohlfasern beträgt zwischen 0,01 mm und 1 mm, insbesondere zwischen 0,1 und 0,5 mm.

Die Gesamtoberfläche der Membran, welche die Kontaktfläche für den Austausch von Substanzen bildet, beträgt zwischen 0,1 und bis zu 10 m², vorzugsweise zwischen 0,2 und 1 m². Das Material, aus dem die Hohlfasern gebildet sind, kann sich aus einer oder mehreren der oben genannten Polymere zusammensetzen, wobei Polymethylpenten oder Poly(organo)siloxan bevorzugt ist. Im Zusammenhang mit der erfindungsgemäßen Anwendung und gegebenenfalls Förderung der Darmflüssigkeit im Betrieb durch den Katheter können die filigranen Hohlfasern der Membran in einer schützenden Katheterhülle untergebracht sein.

Es kann bei der Erfindung vorgesehen sein, dass der Katheter eine Vielzahl von Hohlfasern aufweist, wobei die Fasern jeweils mit einem ersten Anschluss an den Einlass und einem zweiten Anschluss an den Auslass angeschlossen sind, wobei die Hohlfasern bevorzugt als Hohlfaserbündel vorliegen und wobei die Hohlfasern zu einem überwiegenden Teil im Wesentlichen parallel zu einer Längserstreckung des Katheters angeordnet sind.

Gemäß einer weiteren Ausführungsform kann vorgesehen sein, dass die gaspermeable Membran als länglicher Hohlkörper mit einem proximalen und einem distalen Ende ausgebildet ist, wobei die innere Oberfläche der Membran mit dem Trägermedium in Kontakt steht und eine äußere Oberfläche der Membran mit dem Darmtrakt in Kontakt steht. Durch die Ausgestaltung als länglicher Hohlkörper ist der Katheter an die Form des Darms angepasst und erlaubt eine CO₂-Aufnahme ohne die Darmtätigkeit gravierend zu stören.

Der Hohlkörper kann im Betrieb bevorzugt einen Durchmesser von 3 bis 8 cm und weiterhin bevorzugt von 5 bis 7 cm aufweisen. Die Verwendung im Betrieb bedeutet eine Verwendung nach der Positionierung des Katheters und der durch die Zufuhr des Trägermediums erfolgenden Volumenvergrößerung. Der Fachmann wird hier den Durchmesser je nach Darmdurchmesser auswählen, wobei es bevorzugt ist, dass der Katheter bei der Anwendung den Darm überwiegend oder sogar vollständig im Querschnitt ausfüllt, so dass aufgrund des geringen Abstand oder im Extremfall aufgrund des engen Kontakts zwischen Katheter und Darmwand eine maximale Gasdiffusion ermöglicht wird.

Vorteilhafterweise kann bei der Erfindung vorgesehen sein, dass bei dem länglichen Hohlkörper der Einlass am distalen Ende und der Auslass am proximalen Ende angebracht sind, so dass sie bevorzugt eine möglichst langen Strömungspfad definieren. Hierbei tritt das CO₂-aufnehmendes Trägermedium am distalen Ende durch den Einlass in den Hohlkörper ein, durchläuft die gesamte Länge des Hohlkörpers, um am proximalen Ende den Hohlkörper wieder durch den Auslass zu verlassen.

Es kann bei der Erfindung vorgesehen sein, dass der längliche Hohlkörper eine axiale Länge von 3 bis 100 cm aufweist, bevorzugt eine axiale Länge von 20 bis 50 cm und besonders bevorzugt eine axiale Länge von 30 bis 50 cm oder 10 bis 20 cm. Der Fachmann kann hier entsprechend der gewünschten Aufnahmerate und der Größe des Patienten eine passende Länge für den Katheterhohlkörper auswählen.

Gemäß einer weiteren Ausführungsform kann vorgesehen sein, dass der längliche Hohlkörper eine der folgenden Formgestaltungen aufweist:
(a) ein tubusförmiger Hohlkörper mit kreisrundem oder ovalem Querschnitt;
(b) ein Hohlkörper mit längsseitig verlaufenden, im Wesentlichen parallelen Falz- oder Knicklinien;
(c) ein tubusförmiger Hohlkörper mit einem inneren Tubus, der von einem äußeren Tubus umgeben ist, wobei die beiden Tuben einen derart unterschiedlichen Durchmesser aufweisen, dass eine im Wesentlichen zylindrische Passage zwischen den Tuben ausgebildet wird, die längsseitig von dem CO2-aufnehmenden Trägermedium durchströmt werden kann;
(d) ein flächiger Körper mit zwei außengelegenen Membranflächen, die zusammen mit Zwischenwänden Kanäle für das CO₂-aufnehmende Trägermedium bilden, so dass die Membranflächen mit dem Darmtrakt in Kontakt steht und wobei die Membran derart eingerichtet ist, dass sie die Diffusion von Gas aus dem Darmtrakt in den Kanälen zirkulierende CO₂-aufnehmende Trägermedium erlaubt; oder
(e) ein tubusförmiger Hohlkörper, der schraubenförmig gewunden eine Helix bildet, wobei die Helix bevorzugt einen inneren Kanal zur Beförderung des Verdauungsbreis ausbildet.

Bei einer Ausgestaltung als tubusförmiger Hohlkörper mit kreisrundem oder ovalem Querschnitt liegt der Katheter in einer einfach zu realisierenden und dem langgestreckten Darm entsprechende Form vor.

In bevorzugter Weise weist der tubusförmige Hohlkörper dabei einen inneren durchgängigen Kanal zur Beförderung des Verdauungsbreis auf.

Alternativ kann der Hohlkörper mit längsseitig verlaufenden, im Wesentlichen parallelen Falz- oder Knicklinien versehen sein, wodurch die Kontaktoberfläche deutlich vergrößert werden kann.

In einer weiteren Ausführungsform kann als tubusförmiger Hohlkörper mit einem inneren Tubus ausgestaltet sein, wobei der innere Tubus von einem äußeren Tubus umgeben ist, und wobei die beiden Tuben einen derart unterschiedlichen Durchmesser aufweisen, dass eine im Wesentlichen zylindrische Passage zwischen den Tuben ausgebildet wird, die längsseitig von dem CO₂-aufnehmenden Trägermedium durchströmt werden kann. Damit ist gewährleistet, dass das Trägermedium in einer bevorzugt laminaren Strömung nahe an der Außenmembran entlang strömt und dabei in optimaler Weise CO₂ aufnehmen kann.

In einer Ausführungsform ist der Hohlkörper ausgestaltet als ein flächiger Körper mit zwei außengelegenen Membranflächen, die zusammen mit Zwischenwänden Kanäle für das CO₂-aufnehmende Trägermedium bilden, so dass die Membranflächen mit dem Darmmilieu in Kontakt steht und wobei die Membran derart eingerichtet ist, dass sie die Diffusion von Gas aus dem Darmmilieu in den Kanälen zirkulierende CO₂-aufnehmende Trägermedium erlaubt. Durch die flächige Ausgestaltung wird eine große Kontaktoberfläche bereitgestellt. Durch die jeweilige Kanalführung kann das Trägermedium auf unterschiedlichste Weise durch den Hohlkörper geschleust werden und so auch die Pfadlänge variabel angepasst werden. So kann beispielsweise durch eine mäandrierende Kanalführung ein sehr lange Pfadlänge unter optimaler Ausnutzung der vorhandenen Kontaktoberfläche erzielt werden.

Vorteilhafterweise kann bei der Erfindung vorgesehen sein, dass der der Katheter zur Erhöhung der Gasdiffusion in einer der folgenden Formen beweglich ausgestaltet ist:
a) Rotation um die Längsachse;
b) Alternierende Verdrillung eines Hohlfaserbündels;
c) Pulsierende Volumenveränderung.

Gemäß einer Ausführungsform kann vorgesehen sein, dass der Katheter expandierbar oder dilatierbar ausgestaltet ist, so dass er nach Einführen in den Darmtrakt eine Querschnittsvergrößerung erfahren kann. Er kann so beispielsweise in zusammengefalteter oder zusammengerollter Form einfach rektal eingeführt und im Darm positioniert werden und wird dann auf seine finale Ausdehnung expandiert bzw. dilatiert. Dies geschieht in bevorzugter Weise durch Zufuhr des Trägermediums in den Katheter.

Vorteilhafterweise kann bei der Erfindung vorgesehen sein, dass der Einlass und der Auslass gemeinsam als Doppel-Lumen-Schlauch mit der gaspermeablen Membran verbunden sind. Hierbei können der zum Einlass führende und der zum Auslass führende Schlauch benachbart mit einem halbkreisförmigen Querschnitt in einer Side-by-side Konfiguration eines größeren Schlauch vorliegen oder zu einem Schlauchpaar zusammengeschweißt vorliegen. In einer alternativen Ausgestaltung sind die beiden Schlauchlumen koaxial angeordnet.

Gemäß einer weiteren Ausführungsform kann vorgesehen sein, dass das CO₂-aufnehmde Trägermedium vor dem Eintritt in den Katheter mit Sauerstoff angereichert wird und so im Darmtrakt einen CO₂/O₂-Gastaustausch erlaubt. Bei Verwendung eines CO₂-aufnehmenden Trägermediums, das auch Sauerstoff speichert, kann der Katheter auch für einen CO₂/O₂-Gastaustausch verwendet werden. Hierbei muss das CO₂-aufnehmde Trägermedium vor dem Eintritt in den Katheter mit Sauerstoff angereichert werden und kann dann im Darmtrakt sowohl CO₂ aufnehmen als auch O₂ abgeben. Hierdurch kann sowohl die Hyperkapnie als auch die Hypoxie behandelt werden.

Es kann bei der Erfindung vorgesehen sein, dass der Katheter zusätzlich einen Sensor aufweist, der ausgewählt ist aus der Gruppe bestehend aus CO₂-Sensor, Sauerstoffsensor und pH-Sensor. Dem Fachmann sind zahlreiche Ausgestaltungen für einen CO₂-Sensor und auch für einen Sauerstoffsensor oder pH-Sensor bekannt. Durch die Messung des CO₂-Gehalts können die Aufnahmerate des Katheters angepasst werden, indem beispielsweise die Förderrate erhöht oder erniedrigt wird. Bei Verwendung der Metallhydroxide ist das Ausmaß der pH-Veränderung ein Maß für die CO₂-Aufnahmerate, so dass diese in einfacher Weise durch Messung des pH-Werts bestimmt werden kann.

Es kann bei der Erfindung vorgesehen sein, dass der Katheter zusätzlich einen Spülschlauch mit Auslassöffnung in den Darmtrakt aufweist, wobei die Auslassöffnung bevorzugt am proximalen Ende oder nahe des proximalen Endes angebracht ist. Hierdurch kann der Darm während der Behandlung gespült werden und somit der Gasaustausch optimiert werden.

In einer bevorzugten Ausführungsform ist der Katheter als Einwegartikel ausgestaltet. Hierbei ist es zweckmäßig, wenn der Katheter als bei der der Katheter aus einem abgeschlossenen Hohlkörper besteht und im Anschluss an die therapeutische Verwendung im Sinne eines Einmalproduktes verworfen wird.

Vorteilhafterweise kann bei der Erfindung vorgesehen sein, dass der Einlass mit einem Behälter für die CO₂-aufnehmende Trägerflüssigkeit und mit einer Pumpe zum Transport der CO₂-aufnehmenden Trägerflüssigkeit von dem Behälter zum Katheter flüssigkeitsverbunden ist und der Auslass bevorzugt mit einem Auffangbehälter für die Aufnahme des CO₂-aufnehmendes Trägermediums flüssigkeitsverbunden ist. In dieser einfachsten Förderausgestaltung wird das in einem als Reservoir dienenden Behälter vorliegende CO₂-aufnehmende Flüssigkeit mittels Pumpe durch eine erste Flüssigkeitsleitung und den Einlass in den Katheter gepumpt und nach CO₂-Aufnahme auslassseitig durch eine zweite Flüssigkeitsleitung in den Auffangbehälter geleitet.

Gemäß einer weiteren Ausführungsform kann vorgesehen sein, dass der Katheter mit einer extrakorporalen Austauscheinrichtung zur Bildung eines Kreislaufsystems verbunden ist, wobei das Kreislaufsystem eine Pumpe zur Förderung des Trägermediums aufweist, und bevorzugt im Kreislaufsystem zusätzlich eine erste Umschalteinrichtung vorgesehen ist, mit deren Hilfe das aus dem Katheter strömende Trägermedium wahlweise zu dem Katheter zurückgeführt wird, oder dem Auffangbehälter zuleitbar ist. Bei dieser Ausgestaltung liegen also zwei Flüssigkeitspfade nebeneinander vor. Zunächst ein unidirektionaler Pfad gemäß der vorherigen Ausgestaltung und des Weiteren ein Kreislaufsystem, wobei die beiden Flüssigkeitspfade alternativ durch einen Umschalteinrichtung beschickbar sind. So kann das Trägermedium zunächst in dem Kreislaufsystem so lange zirkulieren, bis die gewünschte CO₂-Aufnahmemenge (evtl. sogar bis zu einer CO₂-Sättigung) erfolgt ist, um dann in den Auffangbehälter umgeleitet zu werden, um dann den Katheter anschließend mit neuem Trägermedium zu beschicken.

Als Flüssigkeitsleitung wird zweckmäßigerweise ein flexibler Schlauch verwendet, der im Folgenden auch als Schlauchleitung bezeichnet wird.

Dem Fachmann sind aus dem Gebiet der Katheter hinlänglich Pumpen bekannt, die er in Anpassung an die Fördermenge und Förderrate in geeigneter Weise auswählen kann. Es kann bei der Erfindung vorgesehen sein, dass die Pumpe ausgewählt ist aus der Gruppe bestehend aus Schlauchpumpe, Pulsionspumpe, Zentrifugalpumpe, Membranpumpe und Kolbenpumpe, wobei ein Schlauchpumpe oder eine Zentrifugalpumpe bevorzugt ist.

Vorteilhafterweise kann bei der Erfindung vorgesehen sein, dass die Vorrichtung zusätzlich mit einer Steuerungseinrichtung ausgestattet ist, die bei dem Katheter die Zufuhr oder die Umwälzung des CO₂-aufnehmenden Trägermedium steuert, bevorzugt anhand der CO₂-Aufnahmerate des Trägermediums.

Gemäß einer weiteren Ausführungsform kann vorgesehen sein, dass die Vorrichtung zusätzlich eine Einführungshilfe zum rektalen Einführen des Katheters in den Darmtrakt aufweist. So kann beispielsweise eine Hülse mit einem Hohlraum verwendet werden, wobei der Hohlraum zur Aufnahme des Katheters geeignet ist.

Nach Einführen der Hülse und Positionierung an dem gewünschten Darmabschnitt, kann die Hülse zurückgezogen werden und der Katheter verbleibt an dem Zielort. Hierbei liegt der Katheter bevorzugt in einer leeren bzw. zusammengefalteten Ausgestaltung vor und wird nach Positionierung durch die Beschickung mit dem Trägermedium auf seine finale Größe gebracht.

Alternativ kann die Vorrichtung auch einen Führungsdraht aufweisen, der fest oder reversibel lösbar mit dem Katheter verbunden ist.

In einer weiteren Ausgestaltung kann die Vorrichtung als Einführungshilfe auch eine Aufnahmevorrichtung für ein Endoskop aufweisen
In einem zweiten Aspekt betrifft die Erfindung ein Set, umfassend den erfindungsgemäßen Katheter, eine Pumpe, eine Austauscheinrichtung und einen mit dem Katheter und der Pumpe bzw. Austauscheinrichtung verbundenen Schlauch zum Transport einer Trägerflüssigkeit zwischen dem Katheter und der Pumpe bzw. Austauscheinrichtung.

In einem dritten Aspekt betrifft die Erfindung die erfindungsgemäße Vorrichtung oder das erfindungsgemäße Set zur Verwendung in der Prophylaxe oder Behandlung von Erkrankungen des respiratorischen Systems wie akuten oder chronischen Atemwegserkrankungen oder Lungenerkrankungen; von Herz-Kreislauf-Erkrankungen, metabolischen Entgleisungen wie beispielsweise Ketoazidose, oder von Infektionserkrankungen oder Störungen der Atmung nach schweren Krankheitsverläufen.

Bei der Prophylaxe oder Behandlung von Erkrankungen des respiratorischen Systems kann hierbei vorteilhafterweise auch die Patientensubgruppe der Patienten mit Divertikulose behandelt werden. Bei der Divertikulose sind - meist im Dickdarm (Colon) - eine oder mehrere ballonartige Ausstülpungen (Divertikel) vorhanden. Da der erfindungsgemäße Katheter sicher als singuläre Vorrichtung im Darmlumen positioniert wird, stellen Patienten mit Divertikulose keine Kontraindikation für die Behandlung gemäß des dritten Aspekts der Erfindung dar und diese Patientensubgruppe kann sicher und effektiv mit der erfindungsgemäßen Vorrichtung behandelt werden. Dies ist gerade bei der Notfallbehandlung von großer Bedeutung, da vorab keine Differentialdiagnose auf das Vorliegen von Divertikeln gestellt werden muss.

Vorteilhafterweise kann bei der Erfindung vorgesehen sein, dass die Erkrankung des respiratorischen Systems ausgewählt ist aus der Liste bestehend aus:
(a) chronische obstruktive Lungenerkrankung;
(b) Asthma;
(c) Mukoviszidose;
(d) akutes Lungenversagen;
(e) Hyperkapnie;
(f) Lungenentzündung;
(g) Lungencarcinom;
(h) Lungenfibrose;
(i) Krankheiten der Atemwege nach medizinischen Maßnahmen gemäß ICD-10 J95;
(j) respiratorische Insuffizienz gemäß ICD-10 J96;
(k) sonstige Krankheiten der Atemwege gemäß ICD-10 J97;
(l) Krankheiten der Atemwege bei anderenorts klassifizierten Krankheiten gemäß ICD-10, J99; oder
(m) unreife Lunge.

Bei der Prophylaxe oder Behandlung von Erkrankungen des respiratorischen Systems gemäß den Punkten (a) bis (m) kann auch die Patientensubgruppe der Patienten mit Divertikulose behandelt werden, wie es vorab bereits beschrieben wurde.

In einem weiteren Aspekt betrifft die Erfindung die erfindungsgemäße Vorrichtung oder das erfindungsgemäße Set zur Verwendung in der Induktion einer Hypokapnie in einem Patienten.

Bei der Induktion der Hyperkapnie in einem Patienten kann auch die Patientensubgruppe der Patienten mit Divertikulose behandelt werden, wie es vorab bereits beschrieben wurde.

Es kann bei der Erfindung vorgesehen sein, dass die Verwendung die folgenden Schritte umfasst:
(a) optionale chemische oder mechanische partielle Degradation der Mucosabarriere des Darms zur Erhöhung der Gasdiffusion durch das Darmepithel und/oder Entleerung des Darms durch einen Hebe-Senk-Einlauf;
(b) rektales Einführen des erfindungsgemäßen Katheters zur Positionierung im Lumen des Darmtrakts, bevorzugt im Colon descendens;
(c) extrakorporale Zufuhr von CO₂-aufnehmendem Trägermedium in das Lumen des Katheters zur Aufnahme von CO₂ aus dem Darmtrakt;
(d) optionale Zirkulation des CO₂-aufnehmenden Trägermediums in einem Kreislaufsystem mit extrakorporaler Austauscheinrichtung, bis das Trägermedium als verbrauchtes Trägermedium einen vorgegebenen CO₂-Gehalt aufweist;
(e) Ableiten des verbrauchten Trägermediums aus dem Katheter in einen Auffangbehälter.

Bei der Verwendung kann das CO₂-aufnehmende Trägermedium im Schritt (c) und/oder Schritt (d) bevorzugt mit einer Flussrate von 1 bis 5 L/Min. durch den Katheter geführt werden.

Zweckmäßigerweise enthält das CO₂-aufnehmende Trägermedium einen pH-Indikator. Bei Verwendung der Metallhydroxide ist das Ausmaß der pH-Veränderung ein Maß für die CO₂-Aufnahmerate, so dass die Aufnahmerate in einfacher Weise durch Messung des pH-Werts bestimmt werden kann.

### Definitionen

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst weiterhin Copolymere und so genannte Präpolymere, das heißt reaktive oligomere Voraddukte, deren funktionelle Gruppen am Aufbau von Makromolekülen beteiligt sind.

Der Begriff "Copolymer" bezeichnet im vorliegenden Dokument ein Polymer, das aus zwei oder mehr verschiedenartigen Monomereinheiten zusammengesetzt ist. Damit unterscheidet sich das Copolymer von einem Homopolymer, das aus nur einer (realen oder gedachten) Monomerart aufgebaut ist und entsprechend nur eine Wiederholeinheit besitzt. Copolymere können in fünf Klassen unterteilt werden, nämlich
1.) statistische Copolymere, in denen die Verteilung der beiden Monomeren in der Kette einer statistischen Verteilung folgt,
2.) Gradientcopolymere, die prinzipiell den statistischen Copolymeren ähnlich sind, in denen jedoch der Anteil des einen Monomers im Verlauf der Kette zu- und des anderen abnimmt,
3.) Alternierende Copolymere, in denen sich die beiden Monomere abwechseln,
4.) Blockcopolymere und Segmentcopolymere, die aus längeren Sequenzen oder Blöcken jedes Monomers besteht, und
5.) Pfropfcopolymere (engl.: graft copolymers), bei denen Blöcke eines Monomers auf das Gerüst (Rückgrat) eines anderen Monomers aufgepfropft sind.

Unter dem Begriff "Lösungsmittel" werden im vorliegenden Dokument Verbindungen verstanden, wie sie aufgeführt sind als organische Lösungsmittel in CD Römpp Chemie Lexikon, 9. Auflage, Version 1.0, Georg Thieme Verlag, Stuttgart 1995. Die erfindungsgemäß verwendeten Polyole werden von dieser Definition nicht erfasst, obwohl sie als Lösungsmittel für die Monomere und auch das durch Radikalpolymerisation entstandene niedrigmolekulare Polymer fungieren.

Als "flüssig" werden im vorliegenden Dokument Substanzen verstanden, welche sich verformen lassen und fließfähig sind, was auch hochviskose und pastöse Substanzen einschließt.

Der Begriff Hyperkapnie gemäß der vorliegenden Erfindung beschreibt eine Erhöhung des arteriellen CO₂ -Partialdrucks über 45 mm Hg, und kennzeichnet damit eine pathologisch erhöhte CO₂ -Konzentration im Blut.

Die Ursachen für eine Zunahme des Kohlendioxid-Partialdrucks sind allgemein auf das Versagen der Atempumpe zurückzuführen, wie beispielsweise im Bereich respiratorischer Insuffizienz mit Hypoventilation und daraus resultierender respiratorischer Azidose zu sehen. Als klassische Beispiele sind verlegte Atemwege durch eine zurückgefallene Zunge, sowie durch Schleim und Blut behinderte Atmung zu nennen. Weitere Ursachen für eine Hyperkapnie sind Medikamentenwirkungen (Muskelrelaxantien-Überhang, Opiate, Hypnotika), Störungen der Atemmechanik durch Pneumothorax oder Störungen des Gasaustausches durch ein Lungenödem. Symptome einer Hyperkapnie sind vor allem Bewusstseinseinschränkungen und evtl. Koma (bei starkem Anstieg spricht man auch von einer "Kohlendioxidnarkose").

Der Begriff "Hypokapnie" beschreibt das Gegenteil der Hyperkapnie, also eine Verminderung des arteriellen CO₂-Partialdrucks, respektive eine verminderte CO₂-Konzentration, z.B. durch Hyperventilation entstanden. Hieraus resultiert eine respiratorische Alkalose. Weiterhin kann eine Hypokapnie auch in Folge einer respiratorischen Kompensation einer metabolischen Alkalose auftreten. Kardiozirkulatorische Auswirkungen der Hypokapnie sind ein Anstieg des peripheren Gefäßwiderstandes, eine Abnahme des Herzzeitvolumens, Anstieg des koronaren Widerstandes und eine Abnahme der koronaren Durchblutung.

Es sei ausdrücklich darauf hingewiesen, dass im Rahmen der hier vorliegenden Patentanmeldung unbestimmte Artikel und unbestimmte Zahlenangaben wie "ein...", "zwei..." usw. im Regelfall als mindestens-Angaben zu verstehen sein sollen, also als "mindestens ein...", "mindestens zwei..." usw., sofern sich nicht etwa aus dem Kontext oder dem konkreten Text einer bestimmten Stelle ergibt, dass etwa dort nur "genau ein...", "genau zwei..." usw. gemeint sein soll. Weiterhin sind alle Zahlenangaben sowie Angaben zu Verfahrensparametern und/oder Vorrichtungsparametern im technischen Sinne zu verstehen, d.h. als mit den üblichen Toleranzen versehen zu verstehen. Auch aus der expliziten Angabe der Einschränkung "wenigstens" oder "mindestens" o.ä. darf nicht geschlossen werden, dass bei der einfachen Verwendung von "ein", also ohne die Angabe von "wenigstens" o.ä., ein "genau ein" gemeint ist.

Sofern nicht anders angegeben, handelt es sich im vorliegenden Dokument bei den %-Angaben um Gewichtsprozent-Angaben.

Die hier gezeigten Ausführungsformen stellen nur Beispiele für die vorliegende Erfindung dar und dürfen daher nicht einschränkend verstanden werden. Alternative durch den Fachmann in Erwägung gezogene Ausführungsformen sind gleichermaßen vom Schutzbereich der vorliegenden Erfindung umfasst.

### Ausführungsbeispiele

Verschiedene Ausführungsformen der Erfindung werden im Folgenden anhand der Abbildungen beschrieben. Es zeigt:
- **Fig. 1**: Schematische Darstellung eines als Hohlkörper ausgestalteten erfindungsgemäßen Katheters
- **Fig. 2**: Schematische Darstellung eines als Hohlkörper ausgestalteten erfindungsgemäßen Katheters in Schnittansicht mit proximalem Einlass und distalem Einlass
- **Fig. 3**: Schematische Darstellung eines Hohlfasermembran aufweisenden erfindungsgemäßen Katheters
- **Fig. 4**: Schematische Darstellung eines erfindungsgemäßen Katheters in Schnittansicht mit mäandrierenden Kanälen; sowie
- **Fig. 5**: in schematischer handskizzierter Darstellung einen Katheter und
- **Fig. 6**: dessen Einsatz im Darmtrakt.

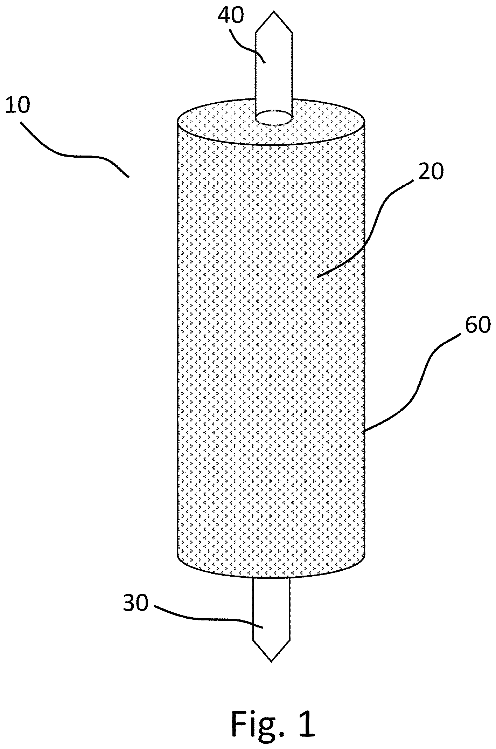

**Figur 1** zeigt die Vorrichtung 10 mit dem Katheter 20 als wesentlichem Bestandteil, der zylinderförmig ausgestaltet ist und durch die gaspermeable Membran 60 umhüllt wird. Der Katheter weist am unteren Ende eine Einlass 30 zur Zufuhr des CO₂-aufnehmenden Trägermediums und am oberen Ende einen Auslass 40 zum Abtransport des CO₂-aufnehmenden Trägermediums nach Aufnahme des CO₂ aus dem Darmmilieu auf.

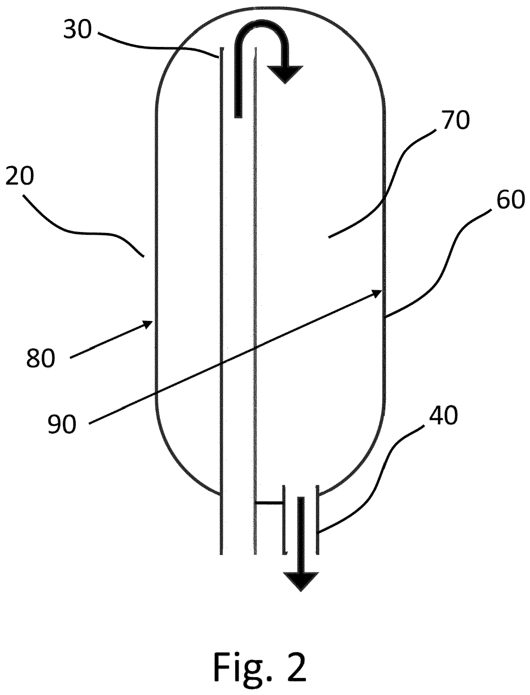

**Figur 2** zeigt eine weitere Ausführungsform für einen erfindungsgemäßen Katheter 20 im Querschnitt, bei dem die gaspermeable Membran 60 einen länglichen Hohlkörper mit abgerundeten Enden bildet und die Membran mit einer ersten Seite 90 mit dem im Lumen 70 befindlichen Trägermedium in Kontakt steht und eine zweite Seite 80 der Membran mit dem Darmmilieu in Kontakt steht und dadurch die Diffusion von Gas aus dem Darmtrakt in das CO₂-aufnehmende Trägermedium erlaubt wird. Der Katheter weist am oberen Ende eine Einlass 30 zur Zufuhr des CO₂-aufnehmenden Trägermediums und am unteren Ende einen Auslass 40 zum Abtransport des CO₂-aufnehmenden Trägermediums nach Aufnahme des CO₂ aus dem Darmmilieu auf.

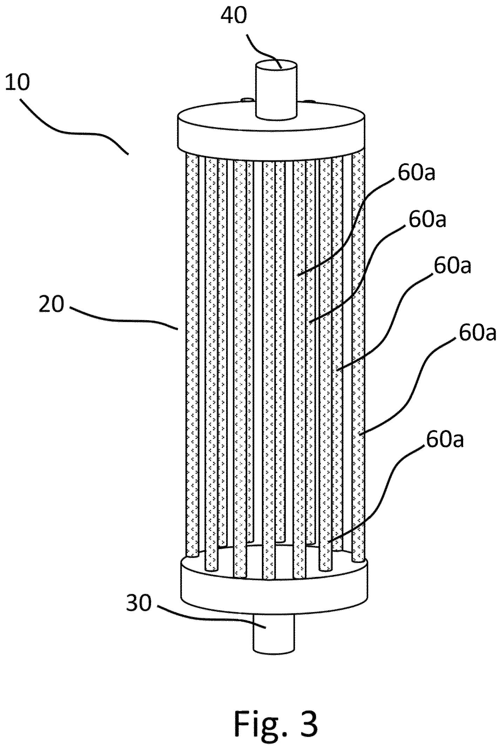

**Figur 3** zeigt die Vorrichtung 10 mit dem Katheter 20 als wesentlichem Bestandteil, der eine Vielzahl von Hohlfasermembranen 60a aufweist, die im Inneren mit Trägermedium durchströmt werden können, und mit der Außenseite der Hohlfasern mit dem Darmmilieu in Kontakt stehen. Der Katheter weist am unteren Ende eine Einlass 30 zur Zufuhr des CO₂-aufnehmenden Trägermediums und am oberen Ende einen Auslass 40 zum Abtransport des CO₂-aufnehmenden Trägermediums nach Aufnahme des CO₂ aus dem Darmmilieu auf.

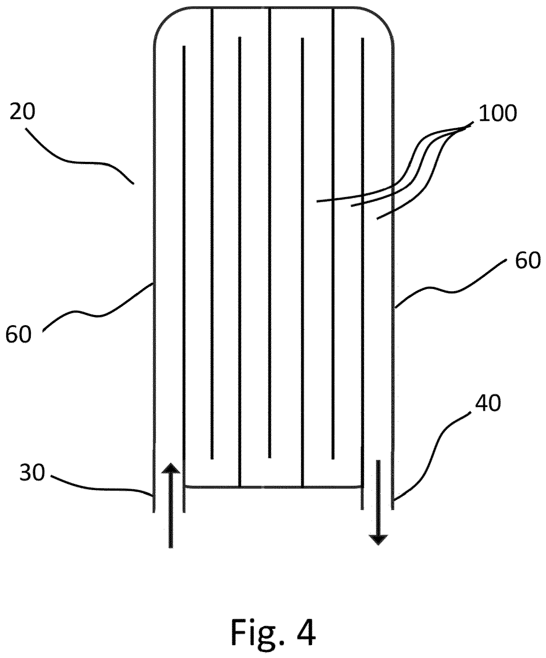

**Figur 4** zeigt die Vorrichtung mit dem Katheter 20 als wesentlichem Bestandteil, der als flächiger Körper ausgestaltet ist und zumindest an den Flachseiten durch die gaspermeable Membran 60 umhüllt wird. Der flächige Körper weist mäandrierende Kanäle 100 auf, durch die das Trägermedium geleitet wird. Der Katheter weist am unteren linken Ende einen Einlass 30 zur Zufuhr des CO₂-aufnehmenden Trägermediums und am unteren rechten Ende einen Auslass zum Abtransport des CO₂-aufnehmenden Trägermediums nach Aufnahme des CO₂ aus dem Darmmilieu auf.

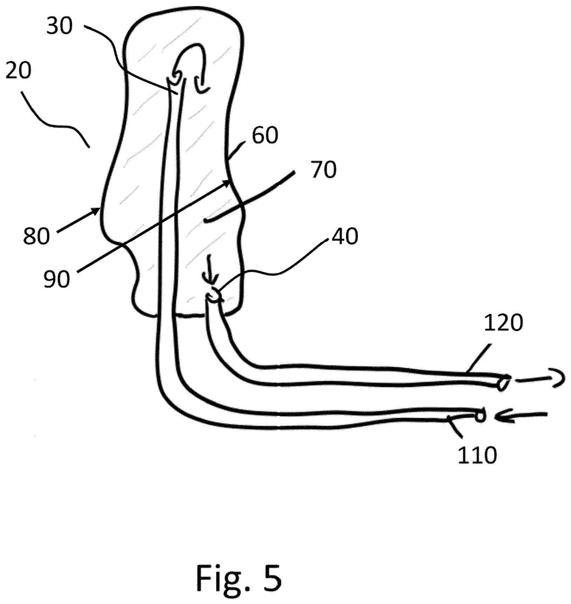

**Figur 5** zeigt eine Ausführungsform für einen erfindungsgemäßen Katheter 20 im Querschnitt, bei dem die gaspermeable Membran 60 einen länglichen beutelförmigen Hohlkörper bildet und die Membran mit einer ersten Seite 90 mit dem im Lumen 70 befindlichen Trägermedium in Kontakt steht und eine zweite Seite 80 der Membran mit dem Darmmilieu in Kontakt steht und dadurch die Diffusion von Gas aus dem Darmtrakt in das im Lumen befindliche, CO₂-aufnehmende Trägermedium erlaubt wird. Der Katheter ist am distalen unteren Ende mit einer ersten zuführenden Schlauchleitung 110 flüssigkeitsverbunden, die im Katheter einen proximal, d.h. am oberen Ende gelegenen Einlass 30 zur Zufuhr des CO₂-aufnehmenden Trägermediums aufweist. Der Katheter ist zudem am distalen, unteren Ende mit einer zweiten abführenden Schlauchleitung 120 flüssigkeitsverbunden, die im Katheter einen distal, d.h. am unteren Ende gelegenen Auslass 40 zum Abtransport des CO₂-aufnehmenden Trägermediums nach Aufnahme des CO₂ aus dem Darmmilieu aufweist.

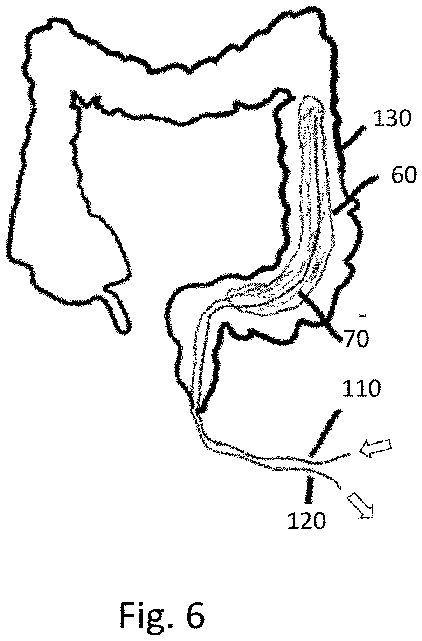

**Figur 6** zeigt in schematischer Weise die therapeutische Anwendung des in Figur 5 dargestellten Katheters. Nach der rektalen Einführung liegt der Katheter im Dick- und Enddarm 130 positioniert vor und ist über die zuführende Schlauchleitung 110 über eine Pumpe mit einem Reservoir für das CO₂-aufnehmende Trägermedium (nicht gezeigt) verbunden. Weiterhin ist der Katheter über die abführende Schlauchleitung 120 mit einem Entsorgungsbehälter als Auffangbehälter (nicht dargestellt) verbunden. Bei der therapeutischen Anwendung befördert die Pumpe das CO₂-aufnehmende Trägermedium über die Schlauchleitung 110 in den Katheter, wo es am proximal gelegenen Einlass in den Katheter eintritt und bei Durchströmen des Membranbeutels Kohlendioxid über die Membran aufnimmt. Das mit CO₂-angereichterte Trägermedium verlässt am proximalen Ende über den Auslass den Katheter und wird über die abführende Schlauchleitung 120 im Auffangbehälter entsorgt.

### Bezugszeichenliste

- 10: Vorrichtung
- 20: Katheter
- 30: Einlass
- 40: Auslass
- 50: Trägermedium
- 60: Gaspermeable Membran
- 60a: Gaspermable Hohlfasermembran
- 70: Lumen
- 80: Außenseite der Membran - Kontakt mit Darmmilieu
- 90: Innenseite der Membran - Kontakt mit Trägermedium
- 100: mäandrierende Kanäle
- 110: zuführender Schlauch
- 120: abführender Schlauch
- 130: Dick- und Enddarm

## Patentansprüche

1. Vorrichtung zum enteralen Aufnehmen von CO₂, aufweisend einen Katheter zur Verwendung im Darmtrakt,
wobei der Katheter
- einen Einlass und
- einen Auslass für ein CO₂-aufnehmendes Trägermedium und
- eine gaspermeable Membran aufweist, wobei
∘ die Membran ein Lumen zur Aufnahme des CO₂-aufnehmenden Trägermediums begrenzt,
∘ wobei eine erste, innere Seite der Membran zum Lumen hin gerichtet ist, sodass sie bei mit dem CO₂-aufnehmenden Trägermedium gefüllten Lumen mit dem Trägermedium in Kontakt steht, und
∘ eine zweite, äußere Seite der Membran zu einer Umgebung hin gerichtet ist, sodass sie bei Positionierung in einem Darm mit dem Darmmilieu in Kontakt steht,
∘ wobei die Membran einen Gasdurchlass von der äußeren zur inneren Seite aufweist, sodass die Membran bei Positionierung im Darmmilieu die Diffusion von Gas aus dem Darmtrakt in das CO₂-aufnehmende Trägermedium erlaubt
und wobei der Katheter mit dem CO₂-aufnehmenden Trägermedium gefüllt ist, **dadurch gekennzeichnet, dass**
das CO₂-aufnehmende Trägermedium eine Flüssigkeit ist, in welcher eine CO₂-absorbierende Substanz dispergiert ist, und
dass Einlass und Auslass separate Zugänge sind.

2. Vorrichtung nach Anspruch 1, **dadurch gekennzeichnet, dass** die gaspermeable Membran wasserundurchlässig ist und/oder einen Molecular weight cutoff (MWCO) von kleiner als 200 Dalton aufweist.

3. Vorrichtung nach Anspruch 1 oder 2, **dadurch gekennzeichnet, dass** die CO₂-absorbierende Substanz eine anorganische Hydroxidverbindung aufweist, die bevorzugt ausgewählt ist aus Lithiumhydroxid, Natriumhydroxid, Kaliumhydroxid, Calciumhydroxid und Magnesiumhydroxid oder Mischungen hiervon, und besonders bevorzugt Magnesiumhydroxid ist.

4. Vorrichtung nach einem der vorangehenden Ansprüche, **dadurch gekennzeichnet, dass** die gaspermeable Membran ein Polymer aufweist, das ausgewählt ist aus der Gruppe bestehend aus Poly(organo)siloxan, Polyethylen wie High-Density-Polyethylen (HDPE) oder Low-Density-Polyethylen (LDPE), Polypropylen, thermoplastisches Polyurethan, Polyester; Polybutylensuccinat; Polybutylenadipat-terephthalat (PBAT), Polyethersulfon (PES), Polyarylethersulfon, Polyacrylethersulfon (PAES), Polyvinylpyrrolidon (PVP), Polymethylmethacrylat (PMMA), Polyamid (PA), Polyacrylnitril (PAN), Ethylen-Vinylalkohol-Copolymer (EVOH), Polytetrafluorethylen, Cellulose, Cellulosetriacetat (CTA), Polymethylpenten (PMP), Cellulosenitrat und Silikon-beschichtetes Polypropylen und Mischungen hiervon, wobei Poly(organo)siloxan besonders bevorzugt ist.

5. Vorrichtung nach einem der vorangehenden Ansprüche, **dadurch gekennzeichnet, dass** die Membran zur Bereitstellung eines gleitfähigen Katheters eine der folgenden Eigenschaften aufweist:
(a) die Membran aus einem gleitfähigen Polymer besteht oder dieses aufweist;
(b) die Membran eine Beschichtung aus einer gleitfähigen Substanz, wie Vaseline, wasserlösliches Gleitgel, PTFE oder Poly(organo)siloxan aufweist; oder
(c) die Membran zum rektalen Einführen mit einem Gleitmittel versehen ist.

6. Vorrichtung nach einem der vorangehenden Ansprüche, **dadurch gekennzeichnet, dass** die Gesamtoberfläche der gaspermeablen Membran, welche die Kontaktoberfläche für die Gasaufnahme bzw. den Gasaustausch bildet, mindestens 0,1 m² beträgt.

7. Vorrichtung nach einem der vorangehenden Ansprüche, **dadurch gekennzeichnet, dass** die gaspermeable Membran als Hohlfasermembran ausgebildet ist, wobei die innere Oberfläche der Hohlfasermembran mit dem Trägermedium in Kontakt steht und die äußere Oberfläche der Hohlfasermembran mit dem Darmtrakt in Kontakt steht, wobei die Hohlfaser bevorzugt einen Durchmesser von weniger als 1 mm aufweist.

8. Vorrichtung nach Anspruch 7, **dadurch gekennzeichnet, dass** der Katheter eine Vielzahl von Hohlfasern aufweist, wobei die Fasern jeweils mit einem ersten Anschluss an den Einlass und einem zweiten Anschluss an den Auslass angeschlossen sind, wobei die Hohlfasern bevorzugt als Hohlfaserbündel vorliegen und wobei die Hohlfasern zu einem überwiegenden Teil im Wesentlichen parallel zu einer Längserstreckung des Katheters angeordnet sind.

9. Vorrichtung nach einem der Ansprüche 1 bis 6, **dadurch gekennzeichnet, dass** die gaspermeable Membran als länglicher Hohlkörper mit einem proximalen und einem distalen Ende ausgebildet ist, wobei die innere Oberfläche der Membran mit dem Trägermedium in Kontakt steht und eine äußere Oberfläche der Membran mit dem Darmtrakt in Kontakt steht, wobei der Hohlkörper im Betrieb bevorzugt einen Durchmesser von 3 bis 8 cm und weiterhin bevorzugt von 5 bis 7 cm aufweist; und/oder wobei bei dem länglichen Hohlkörper der Einlass am distalen Ende und der Auslass am proximalen Ende angebracht sind, sodass sie einen Strömungspfad definieren.

10. Vorrichtung nach einem der Ansprüche 1 bis 6 oder 9, **dadurch gekennzeichnet, dass** der als längliche Hohlkörper ausgebildete Katheter eine der folgenden Formgestaltungen aufweist:
(a) ein tubusförmiger Hohlkörper mit kreisrundem oder ovalem Querschnitt; wobei dieser tubusförmige Hohlkörper bevorzugt einen inneren durchgängigen Kanal zur Beförderung des Verdauungsbreis aufweist;
(b) ein Hohlkörper mit längsseitig verlaufenden, im Wesentlichen parallelen Falz- oder Knicklinien;
(c) ein tubusförmiger Hohlkörper mit einem inneren Tubus, der von einem äußeren Tubus umgeben ist, wobei die beiden Tuben einen derart unterschiedlichen Durchmesser aufweisen, dass eine im Wesentlichen zylindrische Passage zwischen den Tuben ausgebildet wird, die längsseitig von dem CO₂-aufnehmenden Trägermedium durchströmt werden kann;
(d) ein flächiger Körper mit zwei außengelegenen Membranflächen, die zusammen mit Zwischenwänden Kanäle für das CO₂-aufnehmende Trägermedium bilden, so dass die Membranflächen mit dem Darmmilieu in Kontakt steht und wobei die Membran derart eingerichtet ist, dass sie die Diffusion von Gas aus dem Darmmilieu in den Kanälen zirkulierende CO₂-aufnehmende Trägermedium erlaubt; oder
(e) ein tubusförmiger Hohlkörper, der schraubenförmig gewunden eine Helix bildet, wobei die Helix bevorzugt einen inneren Kanal zur Beförderung des Verdauungsbreis aufweist.

11. Vorrichtung nach einem der vorangehenden Ansprüche, **dadurch gekennzeichnet, dass** der Einlass mit einem Behälter für die CO₂-aufnehmende Trägerflüssigkeit und mit einer Pumpe zum Transport der CO₂-aufnehmenden Trägerflüssigkeit von dem Behälter zum Katheter flüssigkeitsverbunden ist und der Auslass bevorzugt mit einem Auffangbehälter für die Aufnahme des CO₂-aufnehmendes Trägermediums flüssigkeitsverbunden ist.

12. Vorrichtung nach einem der vorangehenden Ansprüche, **dadurch gekennzeichnet, dass** der Katheter mit einer extrakorporalen Austauscheinrichtung zur Bildung eines Kreislaufsystems verbunden ist, wobei das Kreislaufsystem eine Pumpe zur Förderung des Trägermediums aufweist, und bevorzugt im Kreislaufsystem zusätzlich eine erste Umschalteinrichtung vorgesehen ist, mit deren Hilfe das aus dem Katheter strömende Trägermedium wahlweise zu dem Katheter zurückgeführt wird, oder dem Auffangbehälter zuleitbar ist.

13. Set umfassend eine Vorrichtung nach 11 oder 12 und einen mit dem Katheter und der Pumpe bzw. Austauscheinrichtung verbundenen Schlauch zum Transport einer Trägerflüssigkeit zwischen dem Katheter und der Pumpe bzw. Austauscheinrichtung.

14. Vorrichtung nach Anspruch 11 oder 12 oder ein Set nach Anspruch 13 geeignet zur Verwendung in der Prophylaxe oder Behandlung von Erkrankungen des respiratorischen Systems wie akuten oder chronischen Atemwegserkrankungen oder Lungenerkrankungen; von Herz-Kreislauf-Erkrankungen, metabolischen Entgleisungen wie beispielsweise Ketoazidose, oder von Infektionserkrankungen oder Störungen der Atmung nach schweren Krankheitsverläufen, bevorzugt zur Verwendung in der Induktion einer Hypokapnie in einem Patienten.

15. Vorrichtung nach Anspruch 14, **dadurch gekennzeichnet, dass** die Verwendung die folgenden Schritte umfasst:
(a) optionale chemische oder mechanische partielle Degradation der Mucosabarriere des Darms zur Erhöhung der Gasdiffusion durch das Darmepithel und/oder eine Entleerung des Darms durch einen Hebe-Senk-Einlauf;
(b) rektales Einführen eines Katheters gemäß einem der Ansprüche 1 bis 10 zur Positionierung im Lumen des Darmtrakts, bevorzugt des Colon descendens;
(c) extrakorporale Zufuhr von CO₂-aufnehmendem Trägermedium in das Lumen des Katheters zur Aufnahme von CO₂ aus dem Darmtrakt, bei Luft bevorzugt 1 bis 5 Liter/Minute, bei einer MgOH2-Suspension bevorzugt um 1 ml/Minute;
(d) optionale Zirkulation des CO₂-aufnehmenden Trägermediums in einem Kreislaufsystem mit extrakorporaler Austauscheinrichtung, bis das Trägermedium als verbrauchtes Trägermedium einen vorgegebenen CO₂-Gehalt aufweist;
(e) Ableiten des verbrauchten Trägermediums aus dem Katheter in einen Auffangbehälter.

## Claims

1. A device for enterally capturing CO₂ comprising a catheter for use in the intestinal tract, wherein the catheter comprises
- an inlet and
- an outlet for a CO₂-capturing carrier medium and
- a gas-permeable membrane, wherein
∘ the membrane defines a lumen for capturing the CO₂-capturing carrier medium,
∘ wherein a first, inner side of the membrane is directed towards the lumen such that it is in contact with the carrier medium when the lumen is filled with the CO₂-capturing carrier medium, and
∘ a second, outer side of the membrane is directed towards an environment such that it is in contact with the intestinal environment when positioned in an intestine,
∘ wherein the membrane comprises a gas passage from the outer to the inner side such that the membrane allows diffusion of gas from the intestinal tract into the CO₂-capturing carrier medium when positioned in the intestinal environment
and wherein the catheter is filled with the CO₂-capturing carrier medium, **characterized in that** the CO₂-capturing carrier medium is a liquid in which a CO₂-absorbing substance is dispersed, and that the inlet and outlet are separate accesses.

2. The device according to claim 1, **characterized in that** the gas-permeable membrane is impermeable to water and/or has a molecular weight cutoff (MWCO) of less than 200 daltons.

3. The device according to claim 1 or 2, **characterized in that** the CO₂-absorbing substance comprises an inorganic hydroxide compound, which is preferably selected from lithium hydroxide, sodium hydroxide, potassium hydroxide, calcium hydroxide and magnesium hydroxide or mixtures thereof, and more preferably is magnesium hydroxide.

4. The device according to any of the preceding claims, **characterized in that** the gas-permeable membrane comprises a polymer selected from the group consisting of poly(organo)siloxane, polyethylene such as high-density polyethylene (HDPE) or low-density polyethylene (LDPE), polypropylene, thermoplastic polyurethane, polyester; polybutylene succinate; polybutylene adipate terephthalate (PBAT), polyethersulfone (PES), polyarylethersulfone, polyacrylethersulfone (PAES), polyvinylpyrrolidone (PVP), polymethylmethacrylate (PMMA), polyamide (PA), polyacrylonitrile (PAN), ethylenevinyl alcohol copolymer (EVOH), polytetrafluoroethylene, cellulose, cellulose triacetate (CTA), polymethylpentene (PMP), cellulose nitrate and silicone-coated polypropylene and mixtures thereof, wherein poly(organo)siloxane is more preferred.

5. The device according to any of the preceding claims, **characterized in that** the membrane comprises one of the following properties for providing a slidable catheter:
(a) the membrane consists of or comprises a slidable polymer;
(b) the membrane comprises a coating of a slidable substance, such as Vaseline, water-soluble sliding gel, PTFE or poly(organo)siloxane; or
(c) the membrane is provided with a lubricant for rectal insertion.

6. The device according to any of the preceding claims, **characterized in that** the total surface area of the gas-permeable membrane, which forms the contact surface for gas capture or gas exchange, is at least 0.1 m².

7. The device according to any of the preceding claims, **characterized in that** the gas-permeable membrane is formed as a hollow fiber membrane, wherein the inner surface of the hollow fiber membrane is in contact with the carrier medium and the outer surface of the hollow fiber membrane is in contact with the intestinal tract, wherein the hollow fiber preferably has a diameter of less than 1 mm.

8. The device according to claim 7, **characterized in that** the catheter comprises a plurality of hollow fibers, wherein the fibers are each connected with a first connection to the inlet and a second connection to the outlet, wherein the hollow fibers are preferably present as hollow fiber bundles and wherein the hollow fibers are arranged for the most part substantially parallel to a longitudinal extension of the catheter.

9. The device according to any of claims 1 to 6, **characterized in that** the gas-permeable membrane is formed as an elongated hollow body with a proximal and a distal end, wherein the inner surface of the membrane is in contact with the carrier medium and an outer surface of the membrane is in contact with the intestinal tract, wherein the hollow body preferably has a diameter of 3 to 8 cm and further preferably of 5 to 7 cm in operation; and/or wherein the inlet at the distal end and the outlet at the proximal end are attached to the elongated hollow body such that they define a flow path.

10. The device according to any of claims 1 to 6 or 9, **characterized in that** the catheter formed as an elongated hollow body comprises one of the following configurations:
(a) a tubular hollow body with a circular or oval cross-section; wherein this tubular hollow body preferably comprises an inner continuous channel for conveying the digestive pulp;
(b) a hollow body with substantially parallel fold or bend lines running on the longitudinal side;
(c) a tubular hollow body with an inner tube which is surrounded by an outer tube, wherein the two tubes have a different diameter such that a substantially cylindrical passage is formed between the tubes, through which passage the CO₂-capturing carrier medium can flow on the longitudinal side;
(d) a planar body with two outer membrane surfaces which together with intermediate walls form channels for the CO₂-capturing carrier medium such that the membrane surfaces are in contact with the intestinal environment and wherein the membrane is adapted such that it allows diffusion of gas from the intestinal environment into the CO₂-capturing carrier medium circulating in the channels; or
(e) a tubular hollow body which forms a helically wound helix, wherein the helix preferably comprises an inner channel for conveying the digestive pulp.

11. The device according to any of the preceding claims, **characterized in that** the inlet is fluidically connected to a container for the CO₂-capturing carrier liquid and to a pump for transporting the CO₂-capturing carrier liquid from the container to the catheter and the outlet is preferably fluidically connected to a collecting container for capturing the CO₂-capturing carrier medium.

12. The device according to any of the preceding claims, **characterized in that** the catheter is connected to an extracorporeal exchange device for forming a circulation system, wherein the circulation system comprises a pump for conveying the carrier medium, and preferably a first switching device is additionally provided in the circulation system, with the aid of which the carrier medium flowing out of the catheter is optionally returned to the catheter or can be fed to the collecting container.

13. A kit comprising a device according to claim 11 or 12 and a tube connected to the catheter and the pump or exchange device for transporting a carrier liquid between the catheter and the pump or exchange device.

14. The device according to claim 11 or 12 or a kit according to claim 13 suitable for use in the prophylaxis or treatment of diseases of the respiratory system such as acute or chronic respiratory diseases or pulmonary diseases; of cardiovascular diseases, metabolic derailments such as, for example, ketoacidosis, or of infectious diseases or disorders of respiration following severe courses of disease, preferably for use in the induction of hypocapnia in a patient.

15. The device according to claim 14, **characterized in that** the use comprises the following steps:
(a) optional chemical or mechanical partial degradation of the mucosa barrier of the intestine for increasing the gas diffusion through the intestinal epithelium and/or emptying of the intestine through a raising-lowering inlet;
(b) rectal insertion of a catheter according to any of claims 1 to 10 for positioning in the lumen of the intestinal tract, preferably of the colon descendent;
(c) extracorporeal supply of CO₂-capturing carrier medium into the lumen of the catheter for capturing CO₂ from the intestinal tract, at air preferably 1 to 5 liters/minute, with a MgOH₂ suspension preferably around 1 ml/minute;
(d) optional circulation of the CO₂-capturing carrier medium in a circulation system with extracorporeal exchange device until the carrier medium has a predetermined CO₂ content as used carrier medium;
(e) discharge of the used carrier medium from the catheter into a collecting container.

## Revendications

1. Dispositif d'absorption entérale de CO₂, comprenant un cathéter destiné à être utilisé dans le tractus intestinal,
dans lequel le cathéter comprend
- une entrée et
- une sortie pour un milieu porteur absorbant le CO₂, et
- une membrane perméable aux gaz, dans lequel
∘ la membrane définit une lumière pour recevoir le milieu porteur absorbant le CO₂,
∘ dans lequel un premier côté interne de la membrane est dirigé vers la lumière de sorte qu'il soit en contact avec le milieu porteur lorsque la lumière est remplie avec le milieu porteur absorbant le CO₂, et
∘ un second côté externe de la membrane est dirigé vers un environnement de sorte qu'il soit en contact avec le milieu intestinal lorsqu'il est positionné dans un intestin,
∘ dans lequel la membrane comprend un passage de gaz du côté externe au côté interne de sorte que la membrane permette la diffusion de gaz du tractus intestinal dans le milieu porteur absorbant le CO₂ lorsqu'il est positionné dans le milieu intestinal,
et dans lequel le cathéter est rempli avec le milieu porteur absorbant le CO₂, **caractérisé en ce que** le milieu porteur absorbant le CO₂ est un liquide dans lequel une substance absorbant le CO₂ est dispersée, et **en ce que** l'entrée et la sortie sont des accès séparés.

2. Dispositif selon la revendication 1, **caractérisé en ce que** la membrane perméable aux gaz est imperméable à l'eau et/ou présente un seuil de masse moléculaire (MWCO) inférieur à 200 daltons.

3. Dispositif selon la revendication 1 ou 2, **caractérisé en ce que** la substance absorbant le CO₂ comprend un composé d'hydroxyde inorganique, qui est de préférence choisi parmi l'hydroxyde de lithium, l'hydroxyde de sodium, l'hydroxyde de potassium, l'hydroxyde de calcium et l'hydroxyde de magnésium ou des mélanges de ceux-ci, et est de manière particulièrement préférée l'hydroxyde de magnésium.

4. Dispositif selon l'une quelconque des revendications précédentes, **caractérisé en ce que** la membrane perméable aux gaz comprend un polymère qui est choisi dans le groupe constitué par le poly(organo)siloxane, le polyéthylène tel que le polyéthylène haute densité (HDPE) ou le polyéthylène basse densité (LDPE), le polypropylène, le polyuréthane thermoplastique, le polyester ; le succinate de polybutylène ; le téréphtalate d'adipate de polybutylène (PBAT), la polyéthersulfone (PES), la polyaryléthersulfone, la polyacryléthersulfone (PAES), la polyvinylpyrrolidone (PVP), le polyméthacrylate de méthyle (PMMA), le polyamide (PA), le polyacrylonitrile (PAN), le copolymère éthylène-alcool vinylique (EVOH), le polytétrafluoroéthylène, la cellulose, le triacétate de cellulose (CTA), le polyméthylpentène (PMP), le nitrate de cellulose et le polypropylène revêtu de silicone et des mélanges de ceux-ci, dans lequel le poly(organo)siloxane est particulièrement préféré.

5. Dispositif selon l'une quelconque des revendications précédentes, **caractérisé en ce que** la membrane présente l'une des propriétés suivantes pour fournir un cathéter lubrifiant :
(a) la membrane est constituée d'un polymère lubrifiant ou comprend celui-ci ;
(b) la membrane comprend un revêtement constitué d'une substance lubrifiante, telle que la vaseline, un gel lubrifiant soluble dans l'eau, le PTFE ou le poly(organo)siloxane ; ou
(c) la membrane est pourvue d'un lubrifiant pour l'introduction rectale.

6. Dispositif selon l'une quelconque des revendications précédentes, **caractérisé en ce que** la surface totale de la membrane perméable aux gaz, qui forme la surface de contact pour l'absorption de gaz ou l'échange de gaz, est d'au moins 0,1 m² .

7. Dispositif selon l'une quelconque des revendications précédentes, **caractérisé en ce que** la membrane perméable aux gaz est réalisée sous forme de membrane à fibres creuses, dans lequel la surface interne de la membrane à fibres creuses est en contact avec le milieu porteur et la surface externe de la membrane à fibres creuses est en contact avec le tractus intestinal, dans lequel la fibre creuse présente de préférence un diamètre inférieur à 1 mm.

8. Dispositif selon la revendication 7, **caractérisé en ce que** le cathéter comprend une pluralité de fibres creuses, dans lequel les fibres sont raccordées à l'entrée à chaque fois par un premier raccord et à la sortie par un deuxième raccord, dans lequel les fibres creuses se présentent de préférence sous forme de faisceaux de fibres creuses et dans lequel les fibres creuses sont disposées pour une majeure partie essentiellement parallèlement à une étendue longitudinale du cathéter.

9. Dispositif selon l'une quelconque des revendications 1 à 6, **caractérisé en ce que** la membrane perméable aux gaz est réalisée sous forme de corps creux allongé avec une extrémité proximale et une extrémité distale, dans lequel la surface interne de la membrane est en contact avec le milieu porteur et une surface externe de la membrane est en contact avec le tractus intestinal, dans lequel le corps creux présente de préférence en fonctionnement un diamètre de 3 à 8 cm et de manière davantage préférée de 5 à 7 cm ; et/ou dans lequel, dans le cas du corps creux allongé, l'entrée est montée au niveau de l'extrémité distale et la sortie est montée au niveau de l'extrémité proximale de sorte qu'elles définissent un trajet d'écoulement.

10. Dispositif selon l'une quelconque des revendications 1 à 6 ou 9, **caractérisé en ce que** le cathéter réalisé sous forme de corps creux allongé présente l'une des configurations suivantes :
(a) un corps creux en forme de tube avec une section transversale circulaire ou ovale ; dans lequel ce corps creux en forme de tube présente de préférence un canal continu interne pour le transport de la suspension digestive ;
(b) un corps creux avec des lignes de pliage ou de pliage essentiellement parallèles s'étendant du côté longitudinal ;
(c) un corps creux en forme de tube avec un tube interne qui est entouré par un tube externe, dans lequel les deux tubes présentent un diamètre différent de telle sorte qu'un passage essentiellement cylindrique soit réalisé entre les tubes, lequel peut être traversé du côté longitudinal par le milieu porteur absorbant le CO₂ ;
(d) un corps plat avec deux surfaces de membrane situées à l'extérieur, qui forment conjointement avec des parois intermédiaires des canaux pour le milieu porteur absorbant le CO₂ de sorte que les surfaces de membrane soient en contact avec le milieu intestinal et dans lequel la membrane est conçue de telle sorte qu'elle permette la diffusion de gaz du milieu intestinal dans le milieu porteur absorbant le CO₂ circulant dans les canaux ; ou
(e) un corps creux en forme de tube qui forme une hélice enroulée en forme d'hélice, dans lequel l'hélice présente de préférence un canal interne pour le transport de la suspension digestive.

11. Dispositif selon l'une quelconque des revendications précédentes, **caractérisé en ce que** l'entrée est en liaison fluidique avec un récipient pour le liquide porteur absorbant le CO₂ et avec une pompe pour le transport du liquide porteur absorbant le CO₂ du récipient au cathéter et la sortie est de préférence en liaison fluidique avec un récipient de collecte pour la réception du milieu porteur absorbant le CO₂.

12. Dispositif selon l'une quelconque des revendications précédentes, **caractérisé en ce que** le cathéter est relié à un dispositif d'échange extracorporel pour former un système de circulation, dans lequel le système de circulation présente une pompe pour le transport du milieu porteur, et de préférence un premier dispositif de commutation est en outre prévu dans le système de circulation, à l'aide duquel le milieu porteur s'écoulant hors du cathéter est renvoyé au choix au cathéter ou peut être acheminé au récipient de collecte.

13. Kit comprenant un dispositif selon la revendication 11 ou 12 et un tuyau relié au cathéter et à la pompe ou au dispositif d'échange pour le transport d'un liquide porteur entre le cathéter et la pompe ou le dispositif d'échange.

14. Dispositif selon la revendication 11 ou 12 ou kit selon la revendication 13 approprié pour l'utilisation dans la prophylaxie ou le traitement de maladies du système respiratoire telles que des maladies des voies respiratoires aiguës ou chroniques ou des maladies pulmonaires ; de maladies cardiovasculaires, de dérives métaboliques telles que par exemple la cétoacidose, ou de maladies infectieuses ou de troubles de la respiration après des évolutions de maladies graves, de préférence pour l'utilisation dans l'induction d'une hypocapnie chez un patient.

15. Dispositif selon la revendication 14, **caractérisé en ce que** l'utilisation comprend les étapes suivantes :
(a) dégradation partielle chimique ou mécanique facultative de la barrière muqueuse de l'intestin pour augmenter la diffusion de gaz à travers l'épithélium intestinal et/ou une vidange de l'intestin par une entrée de levage-abaissement ;
(b) introduction rectale d'un cathéter selon l'une quelconque des revendications 1 à 10 pour le positionnement dans la lumière du tractus intestinal, de préférence du côlon descendant ;
(c) introduction extracorporelle de milieu porteur absorbant le CO₂ dans la lumière du cathéter pour la réception de CO₂ du tractus intestinal, dans l'air de préférence de 1 à 5 litres/minute, avec une suspension de MgOH2 de préférence d'environ 1 ml/minute ;
(d) circulation facultative du milieu porteur absorbant le CO₂ dans un système de circulation avec un dispositif d'échange extracorporel jusqu'à ce que le milieu porteur présente en tant que milieu porteur usé une teneur en CO₂ prédéfinie ;
(e) évacuation du milieu porteur usé hors du cathéter dans un récipient de collecte.
